# EUROPEAN PATENT SPECIFICATION

(11) **EP 1 615 644 B1**
(45) Date of publication and mention of the grant of the patent: **14.02.2007**
(21) Application number: 04726520.2
(22) Date of filing: 08.04.2004
(51) Int. Cl.: A61K 31/454, A61K 31/506, A61K 31/4545, A61K 31/496, A61P 25/22, A61P 25/24, A61P 3/04

(54) **SUBSTITUTED 4-PHENYL-4-[1H-IMIDAZOL-2-YL]-PIPERIDINE DERIVATIVES AND THEIR USE AS SELECTIVE NON-PEPTIDE DELTA OPIOID AGONISTS WITH ANTIDEPRESSANT AND ANXIOLYTIC ACTIVITY**
SUBSTITUIERTE 4-PHENYL-4-[1H-IMIDAZOL-2-YL]-PIPERIDINDERIVATE UND DEREN VERWENDUNG ALS SELEKTIVE NICHT-PEPTIDISCHE DELTA-OPIOID-AGONISTEN MIT ANTIDEPRESSIVER UND ANGSTLÖSENDER AKTIVITÄT
DERIVES 4-PHENYL-4-[1H-IMIDAZOL-2-YL]-PIPERIDINE SUBSTITUES, UTILISATION DE CES DERNIERS EN TANT QU'AGONISTES DELTA OPIOIDES NON PEPTIDIQUES SELECTIFS A ACTIVITE ANTIDEPRESSEUR ET ANXIOLYTIQUE

(30) Priority: 11.04.2003 WO PCT/EP03/03879
(43) Date of publication of application: 18.01.2006
(73) Proprietor: JANSSEN PHARMACEUTICA N.V., 2340 Beerse (BE)
(72) Inventor: STECKLER, Thomas Horst Wolfgang, 2340 Beerse (BE); JANSSENS, Frans Eduard, Janssen Pharmaceutica N.V., 2340 Beerse (BE); LEENAERTS, Joseph Elisabeth, 2340 Beerse (BE); FERNÁNDEZ-GADEA, Francisco J., Janssen Cilag S.A., Campo de las Naciones, 28042 Madrid (ES); GÓMEZ-SÁNCHEZ, Antonio, Johnson & Johnson Pharm.., Poligono Industrial, 45007 Toledo (ES); MEERT, Theo Frans, c/o Janssen Pharmaceutica N.V., 2340 Beerse (BE)
(74) Representative: Van Borm, Werner August H.M.
(86) International application number: PCT/EP2004/050492
(87) International publication number: WO 2004/089372

(56) References cited:
- EP-A- 1 038 872
- WO-A-00/14066
- WO-A-02/094810
- WO-A-03/033486
- WO-A-03/039440
- US-A1- 2002 165 247
- BROOM DANIEL C ET AL: "Behavioral effects of delta-opioid receptor agonists: Potential antidepressants?" JAPANESE JOURNAL OF PHARMACOLOGY, vol. 90, no. 1, September 2002 (2002-09), pages 1-6, XP008026288 ISSN: 0021-5198

## Description

The present invention relates to the use of 4-phenyl-4-[1*H*-imidazol-2-yl]-piperidine derivatives for the treatment of central nervous system disorders, in particular as selective antidepressant and anxiolytic non-peptide δ-opioid agonists.

### Background of the Invention

The presence of at least three populations of opioid receptors (commonly known as mu (µ), delta (δ) and kappa (κ) receptors) is now well established and documented and all three populations appear to be present in the central and peripheral nervous system of many species, including man (Lord J.A.H. et al., *Nature* 1977, 267, 495).

There is an increasing rationale suggesting that endogenous opioids are involved in the response to antidepressant treatment and in stress-related disorders such as depression and anxiety. Endogenous enkephalins have been hypothesized to diminish the impact of stress (Drolet et al., *Prog. Neuro-Psychopharmacol. Biol. Psychiatry* 2001, 25, 729), which could be mediated, at least in part, via activation of δ opioid receptors.

For example, the psychological stress of housing conditions and rank status has been demonstrated to alter the functional activity of δ opioid receptors (Pohorecky et al., *J. Pharmacol. Exp. Ther.* 1999, 290, 196) and, more recently, prenatal stress, which functions as animal model of depression, has been shown to induce a downregulation of δ opioid receptors in different hypothalamic regions in rats (Sanchez et al., *Pharmacology* 2000, 60, 13). Moreover, the monoamine oxidase A inhibitor moclobemide, a clinically active antidepressant drug, increased δ opioid receptor binding in frontal cortex and amygdala after 4 days of treatment in rats (Vilpoux et al., *Eur. J. Pharmacol.* 2002, 443, 85). More support for a possible involvement of δ opioid receptors comes from studies with mouse mutants lacking functional δ opiate receptors, showing anxiogenic- and depressive-like behavioral responses (Filliol et al., *Nature Genet.* 2000, 35, 195). Moreover, enkephalins, as well as enkephalinase inhibitors, such as RB38A, RB38B, RB101 and BL-2401, which block the metabolism of enkephalins, have been reported to attenuate learned helplessness in rats or to produce antidepressant-like effects in forced swimming in mice (Baamonde et al., *Eur. J. Pharmacol.* 1992, 216, 157; Tejedor-Real et al., *Biol. Psychiatry* 1993, 34, 100; *Pharmacol. Biochem. Behav.* 1995, 52, 145; *Eur. J. Pharmacol.* 1998, 354, 1). Similar findings on helpless behavior were reported with the peptidergic δ agonist BUBU (Tejedor-Real et al., *Eur. J. Pharmacol.* 1998, 354, 1). Antidepressant-like activity has also been reported for the selective δ agonist SNC80 and BW373U86 when tested in forced swimming (Broom et al., *Neuropsychopharmacology* 2002, 26, 744). SNC80 also suppresses ultrasonic vocalizations in rats in response to air-puff stress (Pohorecky et al., *J*. *Pharmacol. Exp. Ther.* 1999, 290, 196), providing further evidence for reduced stress responsivity following δ agonism.

Some experiments also suggest that δ-analgesics may also lack the usual side effects associated with µ- and κ-receptor activation (Galligan et al., *J. Pharm. Exp. Ther.* 1984, 229,641).

In view of their important pharmacological value, there is a need for δ-opioid receptor agonists that are selective both in their action as agonists (showing weak or no antagonist action) and for the δ-receptor (showing weak or no preference for the µ- or κ-opioid receptor subtype). Furthermore, such δ-opioid receptor agonists should not be peptidic in nature as such compounds are unstable for administration by systemic routes.

Currently known non-peptidic delta opioid receptor agonists comprise indolo- and benzofuranomorphinans (US-5354863 (1994) by Searle & Co, WO-9531464 (1995) by Astra AB), octahydroisoquinolines (e.g. TAN-67 by Toray Inc., published in JP-4275288 (1992) and WO-9710216 (1997) by Smithkline Beecham SPA), piperazine derivatives (e.g. BW373U86 and SNC 80 by The Welcome Foundation, published in WO-9315062 (1993)), pyrrolooctahydroisoquinolines (WO-9504734 (1995) by Smithkline Beecham SPA), ethylamine derivatives (WO-9622276 (1996) by Nippon Shinyaku Co. Ltd.), triazaspirodecanones (WO 0146192 (2001) by Meiji Seika Kaisha Ltd.) and substituted amino-derivatives (EP-864559 (1998) by Gruenenthal Gmbh).

WO-9828270 (1998) and WO-9828275 (1998) by Astra AB discloses piperidine-derivatives with analgesic activity. Said compounds are not structurally related to the compounds of the present invention.

EP 1 038 872 A1(2000) by Pfizer Products Inc. disclose certain 4-phenyl-4-heteroarylpipcridine derivatives as opioid receptor ligands. Said compounds differ structurally from the ones in the current application - among other - in nature of the piperidinyl nitrogen substitution, which lacks a bivalent π-bond radical substitution and in the substitution of the nitrogens in the imidazolyl-moiety, which are not substituted in EP 1 038 872 A1.

In WO 00/37470 (2000) by Janssen Pharmaceutica N.V. is generally disclosed a pathway for the synthesis of antihistaminic spiro-compounds using some compounds according to the invention. However, said compounds have not exemplified in the prior art application, nor is there any suggestion that they might have δ-opioid receptor agonists properties.

In WO 03/033486 (2003) by Janssen Pharmaceutica N.V. is disclosed the group of compounds of the present invention, as well as their use in the treatment of pain. In WO 03/039440 (2003) by Janssen Pharmaceutica N.V. is disclosed the use of the compounds of the present invention for use in the reduction of ischaemic damage to organs, in particular to reduce cardiac and cerebral ischaemic damage.

### Summary of the Invention

In this application, the use of a group of compounds is described, based on a substituted 4-phenyl-4-[1*H*-imidazol-2-yl]-piperidine derivative, that has important antidepressant-and anxiolytic-like properties.

The object of the present invention is the use of a compound according to Formula (I) 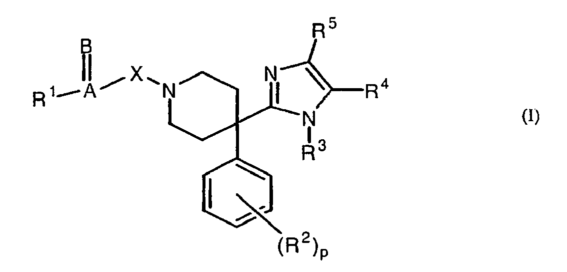 the pharmaceutically acceptable acid or base addition salts thereof, the stereochemically isomeric forms thereof, the tautomeric forms thereof and the N-oxide forms thereof, for the manufacture of a medicament for use in the prevention and/or treatment of central nervous system disorders according to claim 1 wherein:
- A=B: is C=O, C=N-R⁶ wherein R⁶ is hydrogen or cyano, C=S, S=O, SO₂ and C=CR⁷R⁸ wherein R⁷ and R⁸ each independently are hydrogen, nitro or alkyl ;
- X: is a covalent bond, -CH₂- or CH₂CH₂- ;
- R¹: is hydrogen, hydroxy, alkyloxy, alkylcarbonyloxy, Ar-oxy, Het-oxy, Ar-carbonyloxy, Het-carbonyloxy, Ar-alkyloxy, Het-alkyloxy, alkyl, polyhaloalkyl, alkyloxyalkyl, Ar-alkyl, Het-alkyl, Ar, Het, thio, alkylthio, Ar-thio, Het-thio or NR⁹R¹⁰ wherein R⁹ and R¹⁰ each independently are hydrogen, alkyl, Ar,
Ar-alkyl, Het, Het-alkyl, Ar-carbonyl, alkylcarbonyl, Het-carbonyl or alkyloxycarbonylalkyl ;
or A=B and R¹ together form an optionally substituted semi-aromatic or aromatic carbocyclic or heterocyclic radical Het² or Het³ ;
- R²: is hydroxy, alkyloxy, alkylcarbonyloxy, phenyloxy, phenylcarbonyloxy, halo, cyano, alkyl, polyhaloalkyl, alkyloxyalkyl, formyl, carboxy, alkylcarbonyl, alkyloxycarbonyl, aminocarbonyl, mono- or dialkylaminocarbonyl, phenyl, nitro, amino, mono- or dialkyl-amino, thio or alkylthio ;
- R³: is alkyl, Ar, Ar-alkyl, Ar-alkenyl, Ar-carbonyl, Het, Het-alkyl, Het-alkenyl or Het-carbonyl ;
- R⁴, R⁵: each independently is hydrogen, alkyl, carboxy, aminocarbonyl, alkyloxycarbonyl, halo or hydroxyalkyl ;
- p: is an integer equal to zero, 1, 2 or 3 ;

In the framework of this application, alkyl is a straight or branched saturated hydrocarbon radical having from 1 to 6 carbon atoms ; or is a cyclic saturated hydrocarbon (cycloalkyl) radical having from 3 to 7 carbon atoms ; or is a cyclic saturated hydrocarbon radical having from 3 to 7 carbon atoms attached to a straight or branched saturated hydrocarbon radical having from 1 to 6 carbon atoms; wherein each carbon atom may be optionally substituted with amino, nitro, thio, hydroxy, oxo, cyano, formyl or carboxy. Preferably, alkyl is methyl, ethyl, propyl, isopropyl, butyl, tert-butyl, cyclopropyl, cyclopentyl, cyclohexyl, cyclohexylmethyl and cyclohexylethyl.

In the framework of this application, alkenyl is an alkyl radical as defined above having one or more double bonds. Preferably, alkenyl is ethenyl and propenyl.

In the framework of this application, Ar is a homocycle selected from the group of phenyl and naphthyl, each optionally substituted with one or more substituents, each substituent independently selected from the group of hydroxy, alkyloxy, alkylcarbonyloxy, phenyloxy, phenylcarbonyloxy, polyhaloalkyloxy, halo, cyano, alkyl, polyhaloalkyl, alkyloxyalkyl, formyl, haloformyl, carboxy, alkylcarbonyl, alkyloxycarbonyl, aminocarbonyl, mono- or dialkylaminocarbonyl, phenylalkyl, phenyl, nitro, amino, mono- or dialkyl-amino, thio, alkylthio or SO₂-CH₃. Preferably, Ar is naphthyl or phenyl, each optionally substituted with hydroxy, methyloxy, ethyloxy, phenyloxy, trihalomethyloxy, halo, methyl, trifluoromethyl, chloroformyl, carboxy, methyloxycarbonyl, ethyloxycarbonyl, diethylaminocarbonyl, phenyl, nitro, methylthio, trifluoromethyloxy or SO₂-C₁₋₃alkyl.

In the framework of this application, halo is a substituent selected from the group of fluoro, chloro, bromo and iodo and polyhaloalkyl is a straight or branched saturated hydrocarbon radical having from 1 to 6 carbon atoms or a cyclic saturated hydrocarbon radical having from 3 to 7 carbon atoms, wherein one or more carbon atoms is substituted with one or more halo-atoms. Preferably, halo is bromo, fluoro or chloro and preferably, polyhaloalkyl is trifluoromethyl.

In the framework of this application, Het is a heterocyclic radical selected from the group of Het¹, Het² and Het³; wherein each heterocyclic radical Het¹, Het² and Het³ may optionally be substituted on a carbon and/or an heteroatom with halo, hydroxy, alkyloxy, alkyl, Ar, Ar-alkyl or pyridinyl. Het¹ is an aliphatic monocyclic heterocyclic radical selected from the group of pyrrolidinyl, dioxolyl, imidazolidinyl, pyrrazolidinyl, piperidinyl, dioxyl, morpholinyl, dithianyl, thiomorpholinyl, piperazinyl and tetrahydrofuryl. Het² is a semi-aromatic monocyclic heterocyclic radical selected from the group of 2H-pyrrolyl, pyrrolinyl, imidazolinyl and pyrrazolinyl. Het³ is an aromatic monocyclic heterocyclic radical selected from the group of pyrrolyl, pyrazolyl, imidazolyl, furyl, thienyl, oxazolyl, isoxazolyl, thiazolyl, isothiazolyl, pyridinyl, pyrimidinyl, pyrazinyl, pyridazinyl or triazinyl; or an aromatic bicyclic heterocyclic radical selected from the group of quinolinyl, quinoxalinyl, indolyl, benzimidazolyl, benzoxazolyl, benzisoxazolyl, benzothiazolyl, benzisothiazolyl, benzofuranyl and benzothienyl.

### Detailed description of the Invention

An interesting group of compounds are those compounds according to Formula (I), the pharmaceutically acceptable acid or base addition salts thereof, the stereochemically isomeric forms thereof, the tautomeric forms thereof and the N-oxide forms thereof, in which R¹ is selected from the group of alkyloxy, Ar-alkyloxy, alkyl, polyhaloalkyl, alkyloxyalkyl, Ar-alkyl, Het-alkyl, Ar, piperazinyl, pyrrolyl, thiazolyl, pyrrolidinyl and NR⁹R¹⁰ wherein R⁹ and R¹⁰ each independently are hydrogen, alkyl, Ar, Ar-alkyl, pyridinyl or alkyloxycarbonylalkyl.

Another interesting group of compounds are those compounds according to Formula (I), the pharmaceutically acceptable acid or base addition salts thereof, the stereochemically isomeric forms thereof, the tautomeric forms thereof and the N-oxide forms thereof, in which A=B and R¹ together form a radical selected from the group of Het² and Het³. More preferably, A=B and R¹ together form a radical selected from the group of benzoxazolyl, thiazolyl, benzothiazolyl, benzimidazolyl and pyrimidinyl.

Yet another interesting group of compounds are those compounds according to Formula (I), the pharmaceutically acceptable acid or base addition salts thereof, the stereochemically isomeric forms thereof, the tautomeric forms thereof and the N-oxide forms thereof, in which X is a covalent bond or a -CH₂-moiety. Preferably, X is a covalent bond.

Yet another interesting group of compounds are those compounds according to Formula (I), the pharmaceutically acceptable acid or base addition salts thereof, the stereochemically isomeric forms thereof, the tautomeric forms thereof and the *N*-oxide forms thereof, in which R² is alkyloxy or halo.

Yet another interesting group of compounds are those compounds according to Formula (I), the pharmaceutically acceptable acid or base addition salts thereof, the stereochemically isomeric forms thereof, the tautomeric forms thereof and the N-oxide forms thereof, in which R³ is selected from the group of phenylalkyl and naphthyl, each independently substituted with at least one substituent selected from the group of halo, alkyloxycarbonyl, hydroxy, alkyloxy and dialkylaminocarbonyl.

When R³ is alkyl, then preferentially, alkyl is cyclohexylmethyl.

Still another interesting group of compounds are those compounds according to Formula (I), the pharmaceutically acceptable acid or base addition salts thereof, the stereochemically isomeric forms thereof, the tautomeric forms thereof and the N-oxide forms thereof, in which A=B is C=O or SO₂, R¹ is alkyloxy, alkyloxyalkyl, Ar or NR⁹R¹⁰, wherein R⁹ and R¹⁰ each independently are hydrogen or Ar; or A=B and R¹ together form a benzoxazolyl radical ; p is zero, R³ is benzyl optionally substituted with hydroxy, alkyl or alkyloxycarbonyl and R⁴ and R⁵ each are hydrogen.

More specifically, the following compounds are the most preferred compounds :
- 4-[[2-(1-benzoyl-4-phenyl-4-piperidinyl)-1*H*-imidazol-1-yl]methyl]-methylbenzoate ;
- 1-ethoxycarbonyl-4-phenyl-4-[1-(1-phenylethyl)-1*H*-imidazol-2-yl]-piperidine ;
- 4-[[2-[1-(2-benzoxazolyl)-4-phenyl-4-piperidinyl]-1*H*-imidazol-1-yl]methyl]-methylbenzoate ;
- 1-benzoyl-4-phenyl-4-[1-(phenylmethyl)-1*H*-imidazol-2-yl]-piperidine
- 1-benzoyl-4-phenyl-4-[1-(1-phenylethyl)-1*H*-imidazol-2-yl]-piperidine ;
- N,4-diphenyl-4-[1-(phenylmethyl)-1*H*-imidazol-2-yl]-1-piperidinesulfonamide ;
- 1-ethoxycarbonyl-4-phenyl-4-[1-(phenylmethyl)-1*H*-imidazol-2-yl]-piperidine ;
- 1-(methoxyacetyl)-4-phenyl-4-[1-(1-phenylethyl)-1*H*-imidazol-2-yl]-piperidine;
- [4-(1-Benzyl-1*H*-imidazol-2-yl)-4-phenyl-piperidin-1-yl]-(3,5-dimethylphenyl)-methanone ;
- 4-{2-[1-(2-Methoxy-acetyl)-4-phenyl-piperidin-4-yl]-imidazol-1-ylmethyl}-methylbenzoate ;
- 4-(1-Benzyl-1*H*-imidazol-2-yl)-4-phenyl-1-thiazol-2-yl-piperidine;
- 2-{4-Phenyl-4-[1-(1-phenyl-ethyl)-1*H*-imidazol-2-yl]-piperidin-1-yl}-benzo-oxazole ;
- 1-[4-(1-Benzyl-1*H*-imidazol-2-yl)-4-phenyl-piperidin-1-yl]-2-methoxy-ethanone ; and
- 2-[4-(1-Benzyl-1*H*-imidazol-2-yl)-4-phenyl-piperidin-1-yl]-pyrimidine.

The pharmaceutically acceptable acid addition salts are defined to comprise the therapeutically active non-toxic acid addition salt forms which the compounds according to Formula (I) are able to form. Said acid addition salts can be obtained by treating the base form of the compounds according to Formula (I) with appropriate acids, for example inorganic acids, for example hydrohalic acid, in particular hydrochloric acid, hydrobromic acid, sulfuric acid, nitric acid and phosphoric acid ; organic acids, for example acetic acid, hydroxyacetic acid, propanoic acid, lactic acid, pyruvic acid, oxalic acid, malonic acid, succinic acid, maleic acid, fumaric acid, malic acid, tartaric acid, citric acid, mandelic acid, methanesulfonic acid, ethanesulfonic acid, benzenesulfonic acid, p-toluenesulfonic acid, cyclamic acid, salicyclic acid, p-aminosalicylic acid and pamoic acid.

The compounds according to Formula (I) containing acidic protons may also be converted into their therapeutically active non-toxic base addition salt forms by treatment with appropriate organic and inorganic bases. Appropriate base salts forms comprise, for example, the ammonium salts, the alkaline and earth alkaline metal salts, in particular lithium, sodium, potassium, magnesium and calcium salts, salts with organic bases, e.g. the benzathine, *N*-methyl-D-glucamine, hybramine salts, and salts with amino acids, for example arginine and lysine.

Conversely, said acid or base addition salt forms can be converted into the free forms by treatment with an appropriate base or acid.

The term addition salt as used in the framework of this application also comprises the solvates which the compounds according to Formula (I) as well as the salts thereof, are able to form. Such solvates are, for example, hydrates and alcoholates.

The term "stereochemically isomeric forms" as used herein defines all possible isomeric forms which the compounds of Formula (I) may possess. Unless otherwise mentioned or indicated, the chemical designation of compounds denotes the mixture of all possible stereochemically isomeric forms, said mixtures containing all diastereomers and enantiomers of the basic molecular structure. More in particular, stereogenic centers may have the R- or S-configuration; substituents on bivalent cyclic (partially) saturated radicals may have either the cis- or trans-configuration. Stereochemically isomeric forms of the compounds of Formula (I) are obviously intended to be embraced within the scope of this invention.

Following CAS-nomenclature conventions, when two stereogenic centers of known absolute configuration are present in a molecule, an R or S descriptor is assigned (based on Cahn-Ingold-Prelog sequence rule) to the lowest-numbered chiral center, the reference center. The configuration of the second stereogenic center is indicated using relative descriptors [*R**,*R**] or [*R**,*S**], where *R** is always specified as the reference center and [*R**,*R**] indicates centers with the same chirality and [*R**,*S**] indicates centers of unlike chirality. For example, if the lowest-numbered chiral center in the molecule has an *S* configuration and the second center is *R*, the stereo descriptor would be specified as *S*-[*R**,*S**]. If "α" and "β" are used : the position of the highest priority substituent on the asymmetric carbon atom in the ring system having the lowest ring number, is arbitrarily always in the "α" position of the mean plane determined by the ring system. The position of the highest priority substituent on the other asymmetric carbon atom in the ring system relative to the position of the highest priority substituent on the reference atom is denominated "α", if it is on the same side of the mean plane determined by the ring system, or "β", if it is on the other side of the mean plane determined by the ring system.

We note that the substituted carbon atom in the 4-position in the piperidinyl moiety is an achiral atom ; therefore, compounds of Formula (I) may only have at least one stereogenic center in their structure by virtue of a chiral substituent R¹, R², R³, R⁴ or R⁵.

The tautomeric forms of the compounds of Formula (I) are meant to comprise those compounds of Formula (I) wherein e.g. an enol group is converted into a keto group (keto-enol tautomerism).

The *N*-oxide forms of the compounds according to Formula (I) are meant to comprise those compounds of Formula (I) wherein one or several nitrogen atoms are oxidized to the so-called *N*-oxide, particularly those N-oxides wherein the nitrogen of the piperidine moiety and/or the imidazole moiety is oxidized.

The compounds of Formula (I) as prepared in the processes described below may be synthesized in the form of racemic mixtures of enantiomers which can be separated from one another following art-known resolution procedures. The racemic compounds of Formula (I) may be converted into the corresponding diastereomeric salt forms by reaction with a suitable chiral acid. Said diastereomeric salt forms are subsequently separated, for example, by selective or fractional crystallization and the enantiomers are liberated therefrom by alkali. An alternative manner of separating the enantiomeric forms of the compounds of Formula (I) involves liquid chromatography using a chiral stationary phase. Said pure stereochemically isomeric forms may also be derived from the corresponding pure stereochemically isomeric forms of the appropriate starting materials, provided that the reaction occurs stereospecifically. Preferably if a specific stereoisomer is desired, said compound will be synthesized by stereospecific methods of preparation. These methods will advantageously employ enantiomerically pure starting materials.

The compounds according to the invention have surprisingly been shown to have selective delta opioid agonistic activity and show a strong antidepressant and/or anxiolytic activity. In view of their selective delta agonistic activity and their behavioral effects, the compounds according to the invention may be suitable for treatment and/or prophylaxis in the following diseases or any combination thereof :
Central nervous system disorders, selected from:
   Mood disorders, including particularly major depressive disorder, depression with or without psychotic features, catatonic features, melancholic features, atypical features of postpartum onset and, in the case of recurrent episodes, with or without seasonal pattern, dysthymic disorder, bipolar I disorder, bipolar II disorder, cyclothymic disorder, recurrent brief depressive disorder, mixed affective disorder, bipolar disorder not otherwise specified, mood disorder due to a general medical condition, substance-induced mood disorder, mood disorder not otherwise specified, seasonal affective disorder and premenstrual dysphoric disorders.
   Anxiety disorders, including panic attack, agoraphobia, panic disorder without agoraphobia, agoraphobia without history of panic disorder, specific phobia, social phobia, obsessive-compulsive disorder, posttraumatic stress disorder, acute stress disorder, generalized anxiety disorder, anxiety disorder due to a general medical condition, substance- induced anxiety disorder and anxiety disorder not otherwise specified.
   Stress-related disorders associated with depression and/or anxiety, including acute stress reaction, adjustment disorders (brief depressive reaction, prolonged depressive reaction, mixed anxiety and depressive reaction, adjustment disorder with predominant disturbance of other emotions, adjustment disorder with predominant disturbance of conduct, adjustment disorder with mixed disturbance of emotions and conduct, adjustment disorders with other specified predominant symptoms) and other reactions to severe stress.
   Eating disorders, including anorexia nervosa, atypical anorexia nervosa, bulimia nervosa, atypical bulimia nervosa, overeating associated with other psychological disturbances, vomiting associated with other psychological disturbances and non-specified eating disorders.
   Mood disorders induced particularly by alcohol, amphetamines, caffeine, cannabis, cocaine, hallucinogens, inhalants, nicotine, opioids, phencyclidine, sedatives, hypnotics, anxiolytics and other substances.
   Anxiety disorders induced particularly by alcohol, amphetamines, caffeine, cannabis, cocaine, hallucinogens, inhalants, nicotine, opioids, phencyclidine, sedatives, hypnotics, anxiolitics and other substances and adjustment disorders with anxiety.
   *In vitro* receptor and neurotransmitter signal transduction studies can be used to evaluate the delta, mu and kappa opioid receptor agonist activities, as described further in this application.
   Antidepressant- and anxiolytic-like properties are evaluated in rodent models in which antidepressants and anxiolytics are shown to be active. For example, compounds are evaluated in tail suspension, forced swimming and neonatal ultrasonic vocalization.

The compounds according to the invention may be formulated into various pharmaceutical forms for administration purposes. As appropriate compositions there may be cited all compositions usually employed for systemically administering drugs. To prepare the pharmaceutical compositions of this invention, an effective amount of the particular compound, optionally in addition salt form, as the active ingredient is combined in intimate admixture with a pharmaceutically acceptable carrier, which carrier may take a wide variety of forms depending on the form of preparation desired for administration. These pharmaceutical compositions are desirable in unitary dosage form suitable, in particular, for administration by parenteral injection or infusion. For example, in preparing the compositions, any of the usual pharmaceutical media may be employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. Also included are solid form preparations which are intended to be converted, shortly before use, to liquid form preparations.

Depending on the mode of administration, the pharmaceutical composition will preferably comprise from 0.05 to 99 % by weight, more preferably from 0.1 to 70 % by weight of the active ingredient, and, from 1 to 99.95 % by weight, more preferably from 30 to 99.9 weight % of a pharmaceutically acceptable carrier, all percentages being based on the total composition.

The pharmaceutical composition may additionally contain various other ingredients known in the art, for example, a stabilizing agent, buffering agent, emulsifying agent, viscosity-regulating agent, surfactant or preservative.

The present invention also relates to the use of a compound of Formula (I), the pharmaceutically acceptable acid or base addition salts thereof, the stereochemically isomeric forms thereof, the tautomeric forms thereof and the *N*-oxide forms thereof, as well as any of the aforementioned pharmaceutical compositions thereof for the manufacture of a medicament for the treatment of central nervous system disorders, selected from:
Mood disorders, including particularly major depressive disorder, depression with or without psychotic features, catatonic features, melancholic features, atypical features of postpartum onset and, in the case of recurrent episodes, with or without seasonal pattern, dysthymic disorder, bipolar I disorder, bipolar II disorder, cyclothymic disorder, recurrent brief depressive disorder, mixed affective disorder, bipolar disorder not otherwise specified, mood disorder due to a general medical condition, substance-induced mood disorder, mood disorder not otherwise specified, seasonal affective disorder and premenstrual dysphoric disorders.
Mood disorders induced particularly by alcohol, amphetamines, caffeine, cannabis, cocaine, hallucinogens, inhalants, nicotine, opioids, phencyclidine, sedatives, hypnotics, anxiolytics and other substances.
Anxiety disorders, including panic attack, agoraphobia, panic disorder without agoraphobia, agoraphobia without history of panic disorder, specific phobia, social phobia, obsessive-compulsive disorder, posttraumatic stress disorder, acute stress disorder, generalized anxiety disorder, anxiety disorder due to a general medical condition, substance- induced anxiety disorder and anxiety disorder not otherwise specified.
Anxiety disorders induced particularly by alcohol, amphetamines, caffeine, cannabis, cocaine, hallucinogens, inhalants, nicotine, opioids, phencyclidine, sedatives, hypnotics, anxiolitics and other substances and adjustment disorders with anxiety.
Stress-related disorders associated with depression and/or anxiety, including acute stress reaction, adjustment disorders (brief depressive reaction, prolonged depressive reaction, mixed anxiety and depressive reaction, adjustment disorder with predominant disturbance of other emotions, adjustment disorder with predominant disturbance of conduct, adjustment disorder with mixed disturbance of emotions and conduct, adjustment disorders with other specified predominant symptoms) and other reactions to severe stress.
Eating disorders, including anorexia nervosa, atypical anorexia nervosa, bulimia nervosa, atypical bulimia nervosa, overeating associated with other psychological disturbances, vomiting associated with other psychological disturbances and non-specified eating disorders.

The compounds of the present invention may also be co-administered with other antidepressant, antianxiety and/or antipsychotic agents. It will be appreciated that the compounds of the present invention and the other agents may be present as a combined preparation for simultaneous, separate or sequential use for the prevention and/or treatment of central nervous system disorders, in particular depression and/or anxiety. Such combined preparations may be, for example, in the form of a twin pack. It will also be appreciated that the compounds of the present invention and the other agents may be administered as separate pharmaceutical compositions, either simultaneously or sequentially.

Suitable classes of antidepressant agents include norepinephrine reuptake inhibitors, selective serotonin reuptake inhibitors (SSRI's), monoamine oxidase inhibitors (MAOI's), reversible inhibitors of monoamine oxidase (RIMA's), serotonin and noradrenaline reuptake inhibitors (SNRI's), noradrenergic and specific serotonergic antidepressants (NaSSA's), corticotropin releasing factor (CRF) antagonists, α-adrenoreceptor antagonists and atypical antidepressants.

Suitable examples of norepinephrine reuptake inhibitors include amitriptyline, clomipramine, doxepin, imipramine, trimipramine, amoxapine, desipramine, maprotiline, nortriptyline, protriptyline, reboxetine and pharmaceutically acceptable salts thereof.

Suitable examples of selective serotonin reuptake inhibitors include fluoxetine, fluvoxamine, paroxetine, sertraline and pharmaceutically acceptable salts thereof.

Suitable examples of monoamine oxidase inhibitors include isocarboxazid, phenelzine, tranylcypromine, selegiline and pharmaceutically acceptable salts thereof.

Suitable examples of reversible inhibitors of monoamine oxidase include modobemide and pharmaceutically acceptable salts thereof.

Suitable examples of serotonin and noradrenaline reuptake inhibitors include venlafaxine and pharmaceutically acceptable salts thereof.

Suitable atypical antidepressants include bupropion, lithium, nefazodone, trazodone, viloxazine, sibutramine and pharmaceutically acceptable salts thereof.

Other suitable antidepressants include adinazolam, alaproclate, amineptine, amitriptyline/chlordiazepoxide combination, atipamezole, azamianserin, bazinaprine, befuraline, bifemelane, binodaline, bipenamol, brofaromine, bupropion, caroxazone, cericlamine, cianopramine, cimoxatone, citalopram, clemeprol, clovoxamine, dazepinil, deanol, demexiptiline, dibenzepin, dothiepin, droxidopa, enefexine, estazolam, etoperidone, femoxetine, fengabine, fezolamine, fluotracen, idazoxan, indalpine, indeloxazine, iprindole, levoprotiline, litoxetine, lofepramine, medifoxamine, metapramine, metralindole, mianserin, milnacipran, minaprine, mirtazapine, monirelin, nebracctam, nefopam, nialamide, nomifensine, norfluoxetine, orotirelin, oxaflozane, pinazepam, pirlindone, pizotyline, ritanserin, rolipram, sercloremine, setiptiline, sibutramine, sulbutiamine, sulpiride, teniloxazine, thozalinone, thymoliberin, tianeptine, tiflucarbine, tofenacin, tofisopam, toloxatone, tomoxetine, veralipride, viqualine, zimelidine and zometapine and pharmaceutically acceptable salts thereof, and St. John's wort herb, or *Hypericum perforatum,* or extracts thereof.

Suitable classes of anti-anxiety agents include benzodiazepines and 5-HT_{1A} receptor-agonists or antagonists, especially 5-HT_{1A} partial agonists, corticotropin releasing factor (CRF) antagonists, compounds having muscarinic cholinergic activity and compounds acting on ion channels. In addition to benzodiazepines, other suitable classes of anti-anxiety agents are nonbenzodiazepine sedative-hypnotic druges such as zolpidem; mood-stabilizing drugs such as clobazam, gabapentin, lamotrigine, loreclezole, oxcarbamazepine, stiripentol and vigabatrin; and barbiturates.

### Synthesis

The compounds according to the invention can generally be prepared by a succession of steps, each of which is known to the skilled person. In particular, the compounds according to Formula (1-a) can be prepared by reacting an intermediate of Formula (II) according to reaction scheme (1), a reaction that is performed in a suitable reaction-inert solvent, such as toluene, in the presence of a suitable base, such as triethylamine In reaction scheme (I), all variables are defined as in Formula (I) and W¹ together with the moiety it is attached to is equal to R¹ ; examples of W¹ are alkyl, Ar or Het. An example of W¹OC(=O)Cl is chloroformiate.

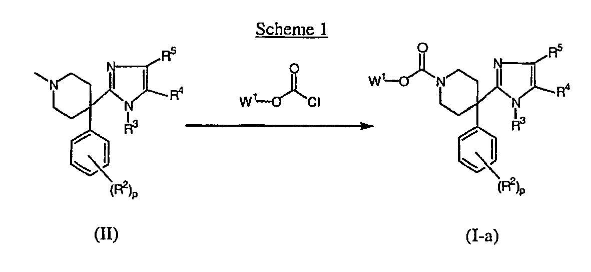

The compounds according to Formulas (I-a), (I-b), (I-c), (I-d), (I-e), (I-f), (I-g), (I-h) and (I-i) can also be prepared by reacting an intermediate of Formula (III) according to any of the reactions shown in reaction scheme (2). In said reactions, all variables are defined as in Formula (I) and W¹ together with the moiety it is attached to is equal to R¹ ; examples of W¹ are alkyl, Ar or Het.
Reaction (a) is performed in a suitable solvent such as dichloroethane and using BOC₂O. The reaction is conveniently carried out for several hours under reflux.
Reaction (b) is performed in a suitable solvent such as THF. The reaction is conveniently carried out for one to several hours at room temperature.
Reaction (c) is performed in a suitable solvent such as dichloromethane in the presence of a suitable base such as Et₃N at room temperature for one hour.
Reaction (d) is performed in a suitable solvent such as THF or DMF at room temperature for several hours with no base needed.
Reaction (e) is performed either in refluxing acetone or in DMF in the presence of a suitable base such as potassium carbonate and can conveniently be carried out at 80°C. Reaction (f) is performed in a suitable solvent such as dichloromethane in the presence of a suitable base such as triethylamine and at room temperature for about 30 to 120 minutes.
Reaction (g) is performed in a suitable solvent such as acetonitril under reflux for 24 hours.
Reaction (h) is performed under different conditions depending on R¹ ; for example when R¹=CF₃ the reaction is performed in the presence of triethylamine in dichloromethane at-78°C for 1 hour. For R¹=NH₂, the reaction is conducted in dioxane for 12 hours at reflux temperature. For R¹=CH₃ the reaction is conducted in dichloromethane at room temperature for 3 hours in the presence of triethylamine. Reaction (i) is performed in a suitable solvent such as isopropanol at reflux temperature for 12-36 hours.
Reaction (j) is performed in a suitable solvent such as acetonitril at reflux temperature for 24 hours.

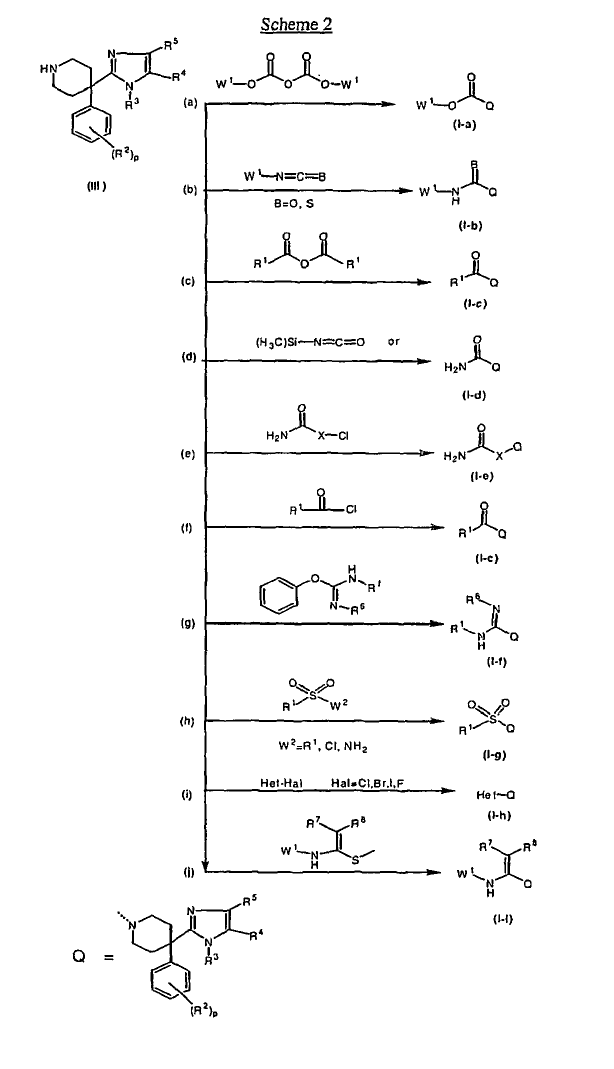

The compounds according to Formulas (I-c) can also be prepared by reacting an intermediate of Formula (IV) with an halide according to the reaction shown in scheme (3). In said reaction, all variables are defined as in Formula (I). The reaction is performed with a base such as NaH (60 % in mineral oil) and in a reaction-inert solvent such as DMF or THF.

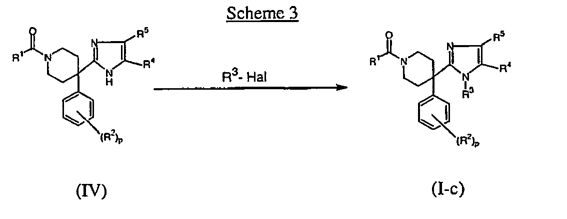

The starting material and the intermediate compounds according to Formulas (II), (III) and (IV) are compounds that are either commercially available or may be prepared according to conventional reaction procedures generally known in the art.

Intermediate compounds of Formula (II) may be prepared according to the following reaction scheme (4) wherein all variables are defined as in Formula (I) :

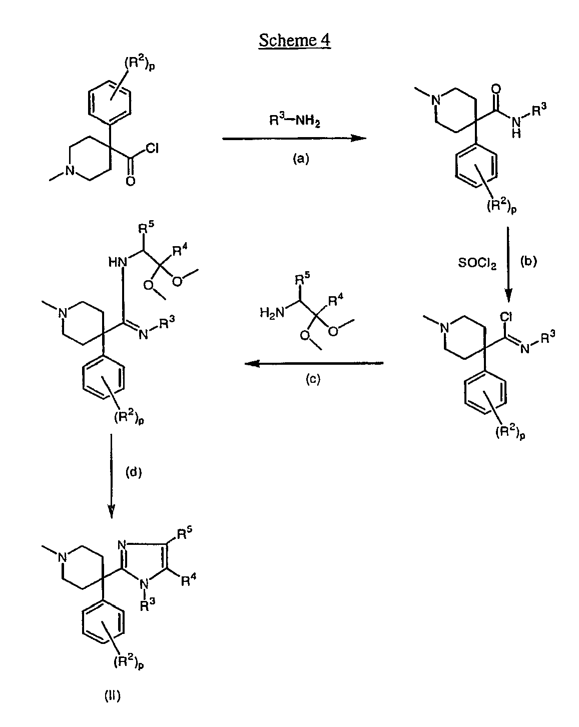

Reaction scheme 4 comprises the step (a) in which an acylchloride of the type shown is reacted with a substituted primary amine, e.g. benzylamine, in the presence of a suitable base, such as Et₃N and in a suitable reaction-inert solvent, such as dichloromethane. The reaction may conveniently carried out at room temperature.
In a next step (b), the adduct obtained in step (a) is refluxed with SOCl₂, after which the product obtained is reacted with appropriately substituted 2,2-dimethoxyethylamine in a reaction-inert solvent, such as DMF, for instance at room temperature (step c). In step (d) the adduct obtained in step (c) is cyclizised in HCl to obtain the substituted imidazolyl-moiety.

Intermediate compounds of Formula (III) may be prepared from compounds according to Formula (I-c) by selectively reducing the R¹-carbonyl-moiety attached to the piperidinyl-moiety according to reaction scheme (5) :,

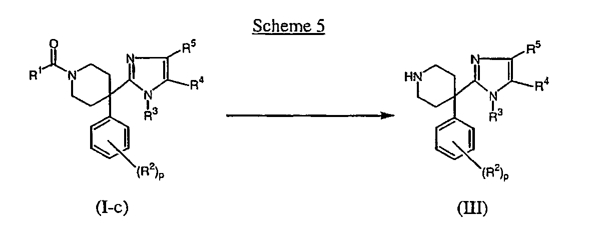

The reaction is performed in the presence of a suitable base, such as KOH, in a suitable reaction-inert solvent, such as 2-propanol and at reflux temperature.

Intermediate compounds according to Formula (IV) may be prepared by hydrogenating compounds according to Formula (1-c) according to reaction scheme (6) : 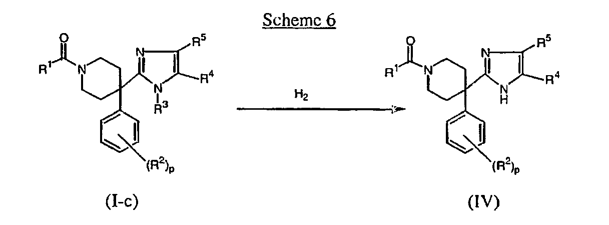 wherein all variables are defined as in Formula (I). The reaction is performed in the presence of a catalyst, such as Pd/C (10 %) in methanol at a moderately elevated temperature.

It is evident that in the foregoing and in the following reactions, the reaction products may be isolated from the reaction medium and, if necessary, further purified according to methodologies generally known in the art, such as extraction, crystallization and chromatography. It is further evident that reaction products that exist in more than one enantiomeric form, may be isolated from their mixture by known techniques, in particular preparative chromatography, such as preparative HPLC.

The following examples illustrate the present invention.

### Experimental part

Of some compounds the absolute stereochemical configuration of the stereogenic carbon atom(s) therein was not experimentally determined. In those cases the stereochemically isomeric form which was first isolated is designated as "A" and the second as "B", without further reference to the actual stereochemical configuration. However, said "A" and "B" isomeric forms can be unambiguously characterized by a person skilled in the art, using art-known methods such as, for example, X-ray diffraction. The isolation method is described in detail below.

Hereinafter, "DMF" is defined as *N,N*-dimethylformamide, 'THF' is defined as tetrahydrofuran and "DIPE" is defined as diisopropyl ether.

### A. Preparation of the intermediate compounds

### Example A1

1-Methyl-4-phenyl-4-piperidinecarbonyl chloride (0.49 mol) was added portionwise at room temperature to a stirred mixture of benzylamine(0.49 mol) and triethylamine (1.223 mol) in CH₂Cl₂ (2500 ml). The mixture was stirred at room temperature for 1 hour. K₂CO₃ (150 g) and H₂O were added. The mixture was stirred and separated into its layers. The aqueous layer was extracted with CH₂Cl₂. The combined organic layer was dried (MgSO₄), filtered and the solvent was evaporated. Yielding: 144 g (95 %) of 1-methyl-4-phenyl-*N*-(phenylmethyl)-4-piperidinecarbox-amide (intermediate compound 1).

### Example A2

A mixture of intermediate compound 1 (0.47 mol) in SOCl₂ (750 ml) was stirred and refluxed for 1 hour. The solvent was evaporated. Toluene was added twice and evaporated again. Yielding: 190 g (100 %) of *N*-[chloro(1-methyl-4-phenyl-4-piperidinyl)methylene]-benzenemethanamine hydrochloride (intermediate compound 2).

### Example A3

A mixture of intermediate compound 2 (0.47 mol) in DMF (750 ml) was cooled on an ice bath. 2,2-Dimethoxyethanamine (0.54 mol) dissolved in DMF was added dropwise. The mixture was stirred at room temperature overnight. The solvent was evaporated. Yielding: 210 g (100 %) of *N*-(2,2-dimethoxyethyl)-1-methyl-4-phenyl-*N*'-(phenylmethyl)-4-piperidinecarboximidamide dihydrochloride (intermediate compound 3).

### Example A4

A mixture of intermediate compound 3 (0.47 mol) in 6N HCl (1500 ml) was stirred until a cloudy solution, then washed with CH₂Cl₂ (900 ml), stirred at 80° C for 1 hour, cooled, alkalized with a NaOH 50 % solution and extracted with CH₂Cl₂. The organic layer was separated, dried (MgSO₄), filtered and the solvent was evaporated. The residue was precipitated from CH₃CN. The precipitate was filtered off and dried. Yielding: 38.3 g (25 %) of 1-methyl-4-phenyl-4-[1-(phenylmethyl)-1*H*-imidazol-2-yl]piperidine (intermediate compound 4).

### Example A5

A mixture of final compound 1 (0.089 mol) in methanol (250 ml) was hydrogenated at 50° C with Pd/C 10 % (3 g) as a catalyst. After uptake of hydrogen (1 equiv.), the catalyst was filtered off and the filtrate was evaporated. The residue was precipitated from CH₃CN. The precipitate was filtered off and dried. Yielding: 23.89 g (90 %) of ethyl-4-phenyl-4-(1*H*-imidazol-2-yl)-1-piperidinecarboxylate (intermediate compound 5).

### Example A6

A mixture of final compound 1 (0.026 mol) and KOH (0.26 mol) in 2-propanol (150 ml) was stirred and refluxed for 10 hours. The solvent was evaporated. The residue was taken up in H₂O and the mixture was extracted with CH₂Cl₂. The organic layer was separated, dried (MgSO₄), filtered and the solvent was evaporated. Yielding: 9.4 g of 4-phenyl-4-[1-(phenylmethyl)-1*H*-imidazol-2-yl]piperidine (intermediate compound 6).

### Example A7

Reaction under N₂ atmosphere. A mixture of intermediate compound 5 (0.0033 mol) in DMF (5 ml) and THF (5 ml) was added dropwise to a solution of NaH, 60 % in mineral oil (0.004 mol) in THF (10 ml), stirred at room temperature. The mixture was stirred for one hour at room temperature. Then, a solution of 4-(acetyloxy)benzylchloride (0.004 mol) in THF was added dropwise and the resulting reaction mixture was refluxed for δ h. After cooling to room temperature, water was added and the resulting mixture was extracted with CH₂Cl₂. The separated organic layer was dried (Na₂SO₄), filtered and the solvent was evaporated. The residue was purified by short open column chromatography over silica gel (eluent: CH₂Cl₂/(CH₃OH/NH₃) 95/5). The pure fractions were collected and the solvent was evaporated. Yielding: 1.33 g of ethyl 4-phenyl-4-[1-((4-methylcarboxy)phenylmethyl)-1*H*-imidazol-2-yl]-1-piperidine-carboxylate (intermediate compound 7).

### B. Preparation of the final compounds

### Example B1

The preparation of final compound 1

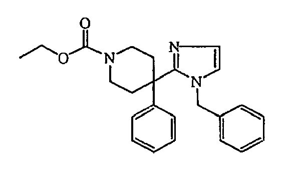

A mixture of intermediate compound 4 (0.05 mol) and N,N-diethylethanamine (0.15 mol) in toluene (750 ml) was stirred at 100 °C. Ethyl chloroformate (0.25 mol) was added dropwise and the reaction mixture was stirred and refluxed for 1 hour and then cooled. The mixture was poured into an aqueous K₂CO₃ solution (35 g K₂CO₃). The layers were separated. The aqueous layer was extracted with CH₂Cl₂. The separated organic layer was dried (MgSO₄), filtered and the solvent evaporated. The residue was purified over silica gel on a glass filter (eluent: CH₂Cl₂/C₂H₅OH 98/2). The desired fractions were collected and the solvent was evaporated. The residue was crystallized from CH₃CN, filtered off and dried. Yielding: 16.7 g (86 %) of ethyl 4-phenyl-4-1-(phenylmethyl)-1*H*-imidazol-2-yl]-1-piperidinecarboxylate (final compound 1).

### Example B2

The preparation of final compound 2

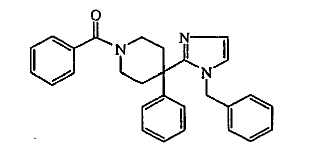

Benzoyl chloride (0.0023 mol) was added to a mixture of intermediate compound 6 (0.0019 mol) and *N*,*N*-diethylethanamine (0.0024 mol) in CH₂Cl₂ (15 ml), stirred at room temperature. The reaction mixture was stirred for 30 min at room temperature. Water was added. The layers were separated. The aqueous layer was extracted with CH₂Cl₂. The combined organic layers were dried (Na₂SO₄), filtered and the solvent evaporated. The residue was purified by short open column chromatography over silica gel (eluent: CH₂Cl₂/(CH₃OH/NH₃) 98/2). The pure fractions were collected and the solvent was evaporated. The residue was recrystallized from n-hexane, filtered off and dried. Yielding: 0.42 g (52 %) of final compound 2; m.p. 122.7° C.

### Example B3

The preparation of final compound 3

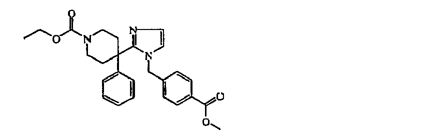

Reaction under N₂ atmosphere. A solution of intermediate compound 5 (0.0054 mol) in DMF (10 ml) and THF (10 ml) was added dropwise to a suspension of NaH (0.00624 mol) in THF (30 ml) and the mixture was stirred at room temperature for 1 hour. Then, methyl 4-(bromomethyl)-benzoate (0.00624 mol) in THF (5 ml) was added dropwise and the reaction mixture was stirred at 60° C for 3 hours. Water was added and the mixture was extracted with CH₂Cl₂. The combined organic layers were dried (Na₂SO₄), filtered and the solvent was evaporated. The residue was purified by short open column chromatography over silica gel (eluent: CH₂Cl₂/(CH₃OH/NH₃) 98/2). The desired fractions were collected and the solvent was evaporated. The residue was crystallized from DIPE, filtered off and dried. Yielding: 1.7 g (70 %) of final compound 3; m.p. 149.1°C.

### Example B4

The preparation of final compound 4

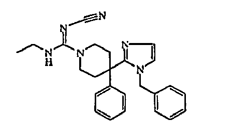

A mixture of intermediate compound 6 (0.0059 mol) and 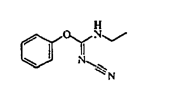 (0.0059 mol) in CH₃CN (70 ml) was stirred and refluxed for 24 hours. The solvent was evaporated. Water was added. The resulting mixture was extracted with CH₂Cl₂. The separated organic layer was dried (Na₂SO₄, anhydrous), filtered and the solvent was evaporated. The residue was crystallized from DIPE, filtered off and recrystallized from CH₃CN, filtered off and dried. Yielding: 0.33 g of final compound 4; m.p. 84.2°C.

### Example B5

The preparation of final compound 5

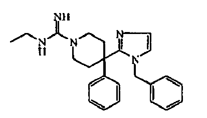

A mixture of final compound 4 (0.0001 mol) in HCl 6N (22.8 ml) was stirred and refluxed for 4 hours. The reaction mixture was alkalized, then extracted with CH₂Cl₂. The separated organic layer was dried (Na₂SO₄, anhydrous), filtered and the solvent was evaporated. The residue was recrystallized from DIPE, filtered off and dried. Yielding: 0.24 g (62 %) of final compound 5.

### Example B6

The preparation of final compound 6

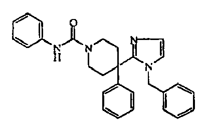

Phenylisocyanate (0.0094 mol) was added dropwise to intermediate compound 6 (0.0094 mol) in THF (50 ml) and the reaction mixture was stirred for 30 min at room temperature. Water was added and this mixture was extracted with CH₂Cl₂. The separated organic layer was dried (Na₂SO₄), filtered and the solvent evaporated. The solid residue was washed with 2-propanone, filtered off and dried. Yielding: 2.7 g (68 %) of final compound 6.

### Example B7

The preparation of final compound 7

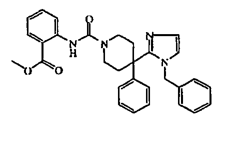

Methyl 2-isocyanatobenzoate (0.0007 mol) was added to intermediate compound 6 (0.0007 mol) in THF (10 ml) and the reaction mixture was stirred for 3 hours at room temperature. Water was added and this mixture was extracted with CH₂Cl₂. The separated organic layer was dried (Na₂SO₄), filtered and the solvent evaporated. The residue (0.4 g) was purified by HPLC over silica gel (eluent: CH₂Cl₂/CH₃OH 98/2). The desired fractions were collected and the solvent was evaporated. Yielding: 0.2 g (66 %) of final compound 7.

### Example B8

a) The preparation of final compound 8 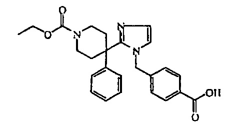
   A mixture of final compound 3 (0.002 mol) and LiOH (0.02 mol) in THF (11 ml) and H₂O (11 ml) was stirred at room temperature for 24 hours. H₂O was added. The mixture was brought to pH 6 and then extracted with CH₂Cl₂. The organic layer was separated, dried (Na₂SO₄), filtered and the solvent was evaporated. The solid residue was washed with CH₂Cl₂, and dried. Yielding: 0.72 g (83 %) of final compound 8; m.p. 251.6°C.
b) The preparation of final compound 9 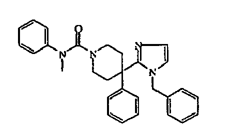
   Reaction under N₂ atmosphere. A suspension of NaH 60 % in mineral oil (0.000642 mol) in DMF (2 ml) was stirred at room temperature. A solution of final compound 6 (0.000642 mol) in DMF (8 ml) was added dropwise and the reaction mixture was stirred for one hour at room temperature. CH₃I (0.001284 mol) was added and the reaction mixture was stirred at 60°C in a pressure vessel for 2 hours. The solvent was evaporated. The residue was purified by high-performance liquid chromatography over silica gel (eluent: CH₂Cl₂/CH₃OH 98/2). The desired fractions were collected and the solvent was evaporated. Yielding: 0.14 g (49 %) of final compound 9.
c) The preparation of final compound 10 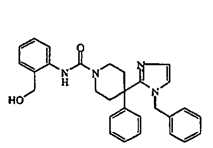
   Reaction under N₂ atmosphere. LiAlH₄ 1 M in THF (0.000444 mol) was added dropwise to a solution of final compound 7 (0.000404 mol) in THF (5 ml), stirred at 0 °C. The reaction mixture was stirred for 30 min at 0 °C. The mixture was treated with a 10 % aqueous NH₄Cl solution and extracted with EtOAc. The separated organic layer was dried (Na₂SO₄), filtered and the solvent evaporated. The residue was purified by CC-TLC on Chromatotron (eluent: CH₂Cl₂/CH₃OH 96/4). The desired fractions were collected and the solvent was evaporated. The residue was crystallized from CH₃OH/H₂O, filtered off and dried. Yielding: 0.020 g (10 %) of final compound 10.
d) The preparation of final compound 11 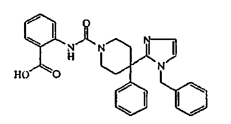
   LiOH (0.001423 mol) was added portionwise to a solution of final compound 7 (0.0006469 mol) in dixoane/H₂O 1/1 (6 ml). The resulting suspension was stirred for 18 hours at room temperature. The solvent was evaporated. The residue was taken up into water and extracted with a mixture of EtOAc and 1-butanol. The organic layer was separated, dried (Na₂SO₄), filtered and the solvent was evaporated. The residue was taken up into 1 N HCl, then extracted with EtOAc. The organic layer was separated, washed with brine, dried (Na₂SO₄), filtered and the solvent was evaporated. The residue was crystallized from Et₂O/CH₂Cl₂, filtered off and dried. Yielding: 0.16 g (51 %) of final compound 11.

### Example B9

LiOH (0.018 mol) was added to a mixture of intermediate compound 7 (0.0018 mol) in THF (10 ml) and H₂O (10 ml). The reaction mixture was stirred for 3 hours at room temperature. Water was added. CH₂Cl₂ was added. The organic layer was separated, dried (Na₂SO₄), filtered and the solvent was evaporated. The white solid residue was washed with methanol and CH₂Cl₂, then dried. Yielding: 0.54 g of ethyl 4-phenyl-4-[1-(4-hydroxyphenylmethyl)-1*H*-imidazol-2-yl]-1-piperidinecarboxylate (final compound 12).

The following compounds as listed in Tables 1-5 were prepared :

**Table 1 :**

| | | | | |
|---|---|---|---|---|
| 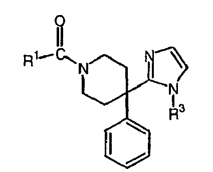 | | | | |

| Comp.nr. | Exp.nr. | R¹ --- | R³--- | Phys.prop. |
|---|---|---|---|---|
| 110 | B2 | --H | 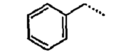 | |
| 13 | B1 | 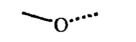 | 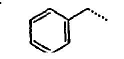 | |
| 14 | B3 | 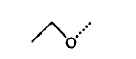 | 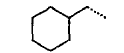 | m.p.=137 |
| 1 | B1 | 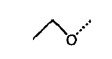 | 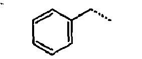 | |
| 12 | B9 | 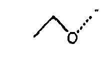 | 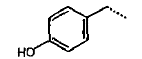 | m.p.=239 |
| 15 | B3 | 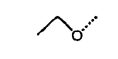 | 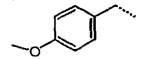 | |
| 16 | B3 | 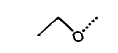 | 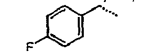 | m.p.=117 |
| 17 | B3 | 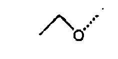 | 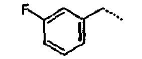 | m.p.=127 |
| 18 | B3 | 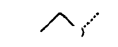 | 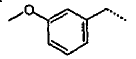 | m.p.=125 |
| 8 | B6 | 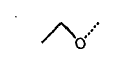 | 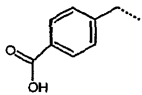 | m.p.=252 |
| 3 | B3 | 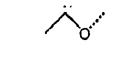 | 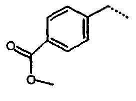 | m.p.=149 |
| 19 | B3 | 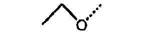 | 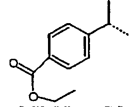 | |
| 20 | B3 | 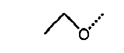 | 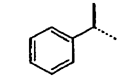 | |
| 21 | B3 | 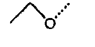 | 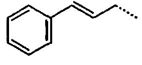 | |
| 22 | B3 | 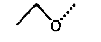 | 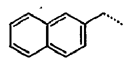 | |
| 23 | B3 | 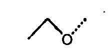 | 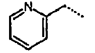 | m.p.=199 |
| 112 | B3 | 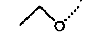 | 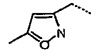 | m.p.=128 |
| 24 | B1 | 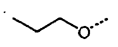 | 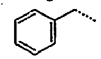 | m.p.=130 |
| 25 | B1 | 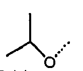 | 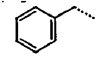 | m.p.=160 |
| 26 | B2 | 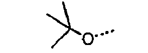 | 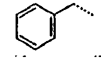 | m.p.=133 |
| 27 | B1 | 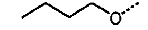 | 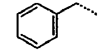 | m.p.=80 |
| 28 | B1 | 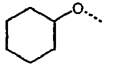 | 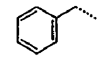 | m.p.=215 |
| 29 | B2 | 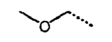 | 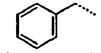 | m.p.=111 |
| 30 | B3 | 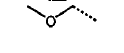 | 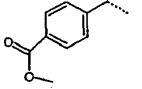 | |
| 31 | B3 | 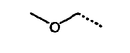 | 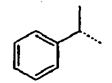 | |
| 32 | B1 | 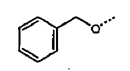 | 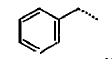 | |
| 33 | B2 | CH₃--- | 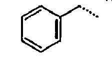 | m.p.=183 |
| 34 | B2 | CH₃CH₂--- | 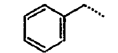 | m.p.=133 |
| 35 | B2 | isopropyl--- | 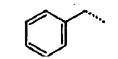 | m.p.=107 |
| 36 | B2 | 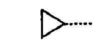 | 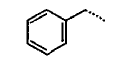 | m.p.=111 |
| 37 | B2 | tert-butyl--- | 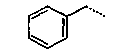 | m.p.=165 |
| 2 | B2 | 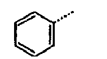 | 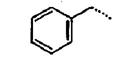 | m.p.=123 |
| 38 | B3 | 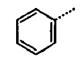 | 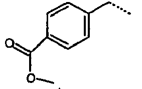 | |
| 39 | B3 | 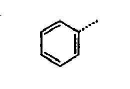 | 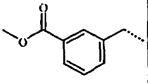 | |
| 40 | B3 | 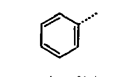 | 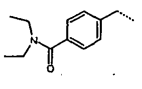 | |
| 41 | B3 | 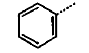 | 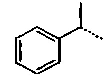 | |
| 42 | B2 | 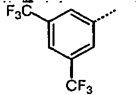 | 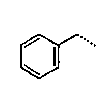 | m.p.=151 |
| 43 | B2 | 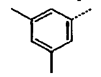 | 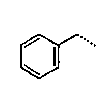 | m.p.=79 |
| 44 | B2 | 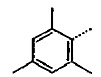 | 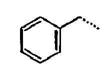 | m.p.=149 |
| 45 | B2 | 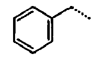 | 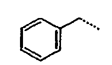 | |
| 46 | B2 | NH₂--- | 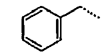 | m.p.=208 |
| 47 | B2 | 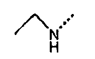 | 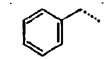 | m.p.=144 |
| 48 | B2 | 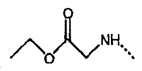 | 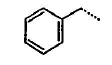 | |
| 49 | B2 | 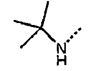 | 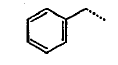 | |
| 50 | B2 | 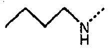 | 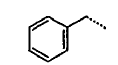 | |
| 51 | B2 | 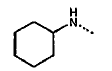 | 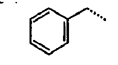 | |
| 6 | B6 | 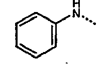 | 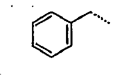 | |
| 52 | B3 | 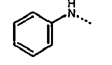 | 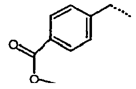 | |
| 53 | B3 | 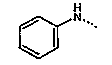 | 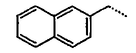 | |
| 54 | B3 | 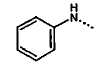 | 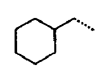 | |
| 55 | B3 | 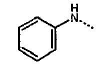 | 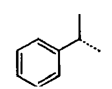 | |
| 56 | B3 | 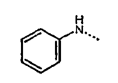 | 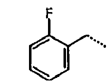 | |
| 57 | B3 | 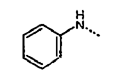 | 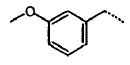 | |
| 58 | B3 | 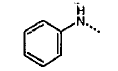 | 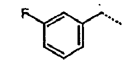 | |
| 59 | B3 | 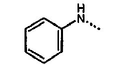 | 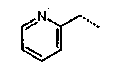 | |
| 60 | B3 | 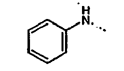 | 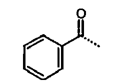 | |
| 61 | B3 | 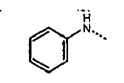 | 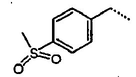 | |
| 62 | B3 | 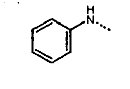 | 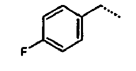 | |
| 63 | B3 | 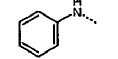 | 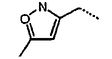 | |
| 64 | B2 | 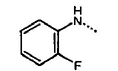 | 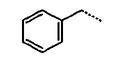 | |
| 65 | B2 | 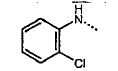 | 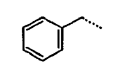 | |
| 66 | B2 | 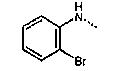 | 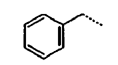 | |
| 67 | B2 | 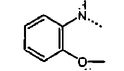 | 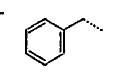 | |
| 68 | B2 | 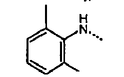 | 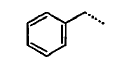 | |
| 7 | B7 | 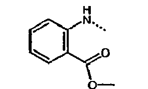 | 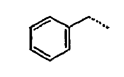 | |
| 69 | B2 | 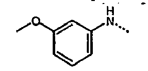 | 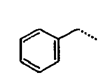 | |
| 117 | B2 | 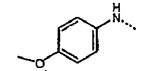 | 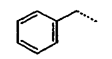 | |
| 70 | B2 | 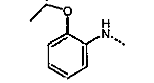 | 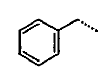 | |
| 71 | B2 | 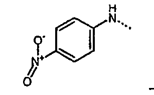 | 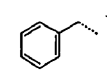 | |
| 72 | B2 | 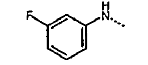 | 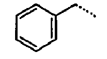 | |
| 73 | B2 | 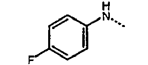 | 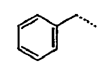 | |
| 74 | B2 | 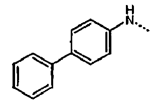 | 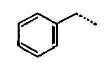 | |
| 10 | B6 | 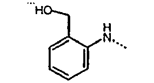 | 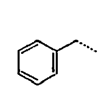 | |
| 75 | B2 | 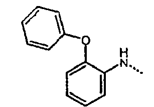 | 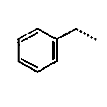 | |
| 76 | B2 | 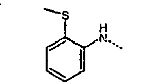 | 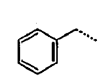 | |
| 77 | B2 | 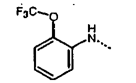 | 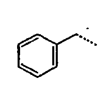 | |
| 11 | B6 | 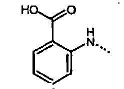 | 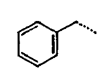 | m.p.=160 |
| 78 | B2 | 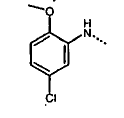 | 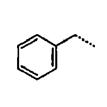 | |
| 79 | B2 | 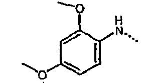 | 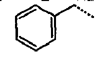 | |
| 9 | B6 | 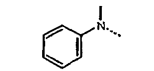 | 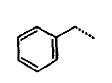 | |
| 80 | B2 | 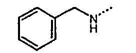 | 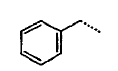 | |
| 81 | B2 | 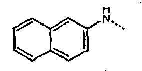 | 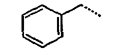 | |
| 113 | B2 | 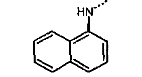 | 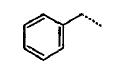 | |
| 82 | B2 | 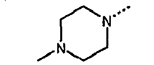 | 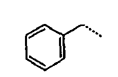 | |
| 83 | B2 | 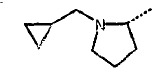 | 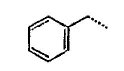 | m.p.=74 |
| 84 | B2 | 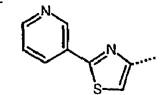 | 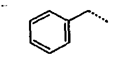 | |
| 85 | B2 | 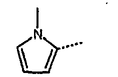 | 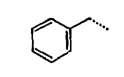 | m.p.=165 |
| 86 | B2 | 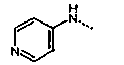 | 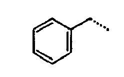 | |
| 87 | B2 | 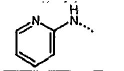 | 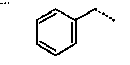 | |

**Table 2 :**

| | | | | | | |
|---|---|---|---|---|---|---|
| 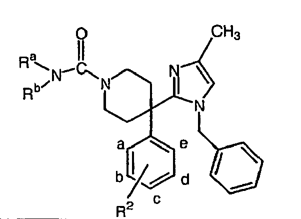 | | | | | | |

| Comp. nr. | Exp. nr. | R^{a}--- | R^{b}--- | R² --- | Position of R² | Phys.data |
|---|---|---|---|---|---|---|
| 88 | B3 | 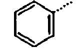 | H | 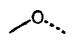 | c | |
| 89 | B3 | 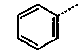 | H | ---F | c | |
| 90 | B3 | 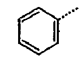 | H | ---F | a | |
| 114 | B3 | 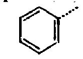 | 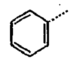 | - | - | |
| 115 | B3 | 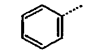 | H | - | - | |

**Table 3:**

| | | | | |
|---|---|---|---|---|
| 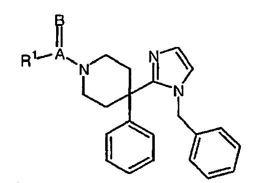 | | | | |

| Comp. nr. | Exp. nr. | A=B | R^{1.....} | Phys.data |
|---|---|---|---|---|
| 5 | B5 | C=NH | 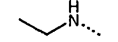 | |
| 91 | B5 | C=N-H | 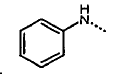 | |
| 4 | B4 | C=N-CN | 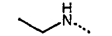 | m.p.=84 |
| 92 | B4 | C=N-CN | 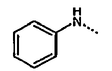 | |
| 93 | B4 | C=C-NO₂ | 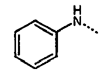 | |
| 95 | B2 | C=S | 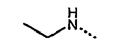 | m.p.=172 |
| 96 | B2 | C=S | 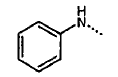 | |
| 94 | B2 | SO₂ | ---CH₃ | m.p. = 167 |
| 97 | B2 | SO₂ | ---NH₂ | m.p.=212 |
| 111 | B2 | SO₂ | ---CF₃ | m.p.=104 |
| 98 | B2 | SO₂ | 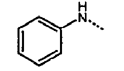 | |

**Table 4:**

| | | | | |
|---|---|---|---|---|
| 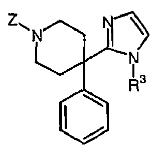 | | | | |

| Comp. nr. | Exp. nr. | Z (A=B and R¹ together) | R^{3.....} | Phys.data |
|---|---|---|---|---|
| 99 | B3 | 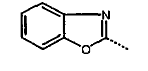 | 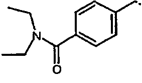 | |
| 100 | B3 | 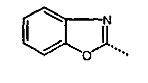 | 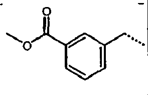 | |
| 101 | B3 | 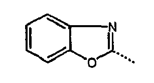 | 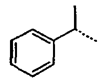 | |
| 102 | B3 | 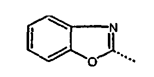 | 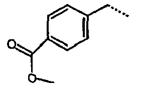 | |
| 103 | B2 | 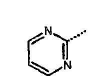 | 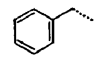 | m.p.=204 |
| 104 | B2 | 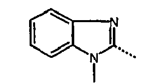 | 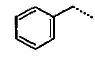 | m.p.=181 |
| 105 | B2 | 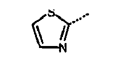 | 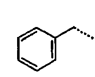 | m.p.=190 |
| 106 | B2 | 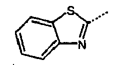 | 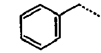 | m.p.=107 |
| 107 | B3 | 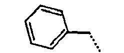 | 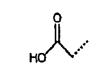 | |

**Table 5:**

| | | | |
|---|---|---|---|
| 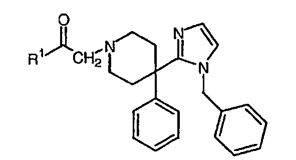 | | | |

| Comp. nr. | Exp. nr. | R^{1 .....} | Phys.data |
|---|---|---|---|
| 108 | B2 | 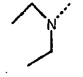 | m.p.=105 |
| 109 | B2 | ---NH₂ | m.p.=136 |

### C. Analytical data

For most of the compounds, either melting points or LCMS data were recorded. The LCMS data are summarized in Table 6.

### LCMS conditions

The HPLC gradient was supplied by a Waters Alliance HT 2790 system with a columnheater set at 40 °C. Flow from the column was split to a Waters 996 photodiode array (PDA) detector and a Waters-Micromass ZQ mass spectrometer with an electrospray ionization source operated in positive and negative ionization mode. Reversed phase HPLC was carried out on a Xterra MS C18 column (3.5 µm, 4.6 x 100 mm) with a flow rate of 1.6 ml/min. Three mobile phases (mobile phase A 95 % 25 mM ammonium acetate + 5 % acetonitrile; mobile phase B: acetonitrile; mobile phase C: methanol) were employed to run a condition from 100 % A to 50 % B and 50 % C in 6.5 min., to 100 % B in 1 min, 100 % B for 1 min. and reequilibrate with 100 % A for 1.5 min. An injection volume of 10 µL was used.

Mass spectra were acquired by scanning from 100 to 1000 in 1 s using a dwell time of 0.1 s. The capillary needle voltage was 3 kV and the source temperature was maintained at 140 °C . Nitrogen was used a the nebulizer gas. Cone voltage was 10 V for positive ionzation mode and 20 V for negative ionization mode. Data acquisition was performed with a Waters-Micromass MassLynx-Openlynx data system.

**Table 6 : LCMS parent peak and retention time for selected compounds.**

| **Comp. no.** | **Retention time** | **LCMS MS(MH+)** |
|---|---|---|
| 2 | 5.57 | 422 |
| 5 | 4.32 | 388 |
| 6 | 5.49 | 437 |
| 7 | 6.15 | 495 |
| 9 | 5.63 | 451 |
| 13 | 5.43 | 376 |
| 15 | 5.49 | 420 |
| 19 | 5.97 | 476 |
| 21 | 5.58 | 416 |
| 22 | 5.71 | 440 |
| 29 | 4.91 | 390 |
| 30 | 4.77 | 448 |
| 31 | 4.82 | 404 |
| 32 | 6.07 | 452 |
| 38 | 5.43 | 480 |
| 39 | 5.42 | 480 |
| 40 | 5.25 | 521 |
| 41 | 5.73 | 436 |
| 48 | 5.03 | 447 |
| 49 | 5.53 | 417 |
| 50 | 5.47 | 417 |
| 51 | 5.71 | 443 |
| 52 | 5.36 | 495 |
| 53 | 5.6 | 487 |
| 54 | 6.01 | 443 |
| 55 | 5.37 | 451 |
| 56 | 5.51 | 455 |
| 57 | 5.11 | 467 |
| 58 | 5.48 | 455 |
| 59 | 4.74 | 438 |
| 60 | 4.61 | 451 |
| 61 | 4.62 | 515 |
| 62 | 5.5 | 455 |
| 63 | 4.86 | 442 |
| 64 | 5.49 | 455 |
| 65 | 5.78 | 471 |
| 66 | 4.63 | 515 |
| 67 | 5.69 | 467 |
| 68 | 5.55 | 465 |
| 69 | 5.51 | 467 |
| 70 | 5.94 | 481 |
| 71 | 5.67 | 482 |
| 72 | 5.64 | 455 |
| 74 | 6.09 | 513 |
| 77 | 5.43 | 521 |
| 78 | 6.03 | 501 |
| 79 | 5.55 | 497 |
| 80 | 5.48 | 451 |
| 81 | 5.91 | 487 |
| 82 | 4.96 | 444 |
| 84 | 5.4 | 506 |
| 86 | 5.04 | 438 |
| 87 | 5.3 | 438 |
| 88 | 5.39 | 467 |
| 89 | 5.48 | 455 |
| 90 | 5.36 | 455 |
| 91 | 4.66 | 436 |
| 92 | 5.42 | 461 |
| 93 | 5.44 | 480 |
| 96 | 5.63 | 453 |
| 98 | 5.68 | 473 |
| 99 | 5.84 | 534 |
| 100 | 5.98 | 493 |
| 101 | 6.27 | 449 |
| 107 | 2.77 | 376 |
| 110 | 4.79 | 346 |
| 117 | 5.41 | 467 |

### D. Pharmacological examples

The pharmacological properties were examined for radioligand binding as well as GTPγS binding assays of the selected compounds on the cloned human δ, κ and µ opioid receptors, expressed in a mammalian cell line. Second messenger signaling was measured on membrane preparations via stimulation of [³⁵S]GTPγS binding. In this functional assay, agonistic and antagonistic properties of the compounds were investigated. DPDPE ((D-Pen ^{2,5})enkephalin) was used as the reference agonist and naltrindole as the reference antagonist for the δ opioid receptor (*Malatynska E., et al.: Human δ opioid receptor: a stable cell line for functional studies of opioids. NeuroReport 6, 613-616, 1995 ; Portoghese P.S. et al., Naltrindole, a highly selective and potent non-peptide δ opioid receptor antagonist. Eur. J. Pharmacol. 146, 185-186, 1988*) and U69593 and nor-binaltorphimine (nor-BNI) were used for the κ opioid receptor as the reference agonist and antagonist, respectively. For the *µ* opioid receptor, morphine was used as the reference agonist and naloxone as the reference antagonist (*Alt A. et al*., *Stimulation of guanosine-5'-O-(3-[³⁵S]thio)triphosphate binding by endogenous opioids acting at a cloned Mu receptor. J. Pharmacol. Exp. Ther. 286, 282-288, 1998 ; Smart D. et al., The effects of recombinant rat µ-opioid receptor activation in CHO cells on phospholipase C, [Ca²⁺]I and adenylyl cyclase. Br. J. Pharmacol. 120, 1165-1171, 1997).*

### Materials and methods

### Cell culture

CHO cells, permanent transfected with the *κ* or *µ* opioid receptor, were cultured in Dulbecco's modified Eagle's medium (DMEM)/ Nutrient mixture Ham's F12 (ratio 1:1) supplemented with 10% heat inactivated fetal calf serum, and an antibiotic solution containing 100 IU/ml penicillin G, 100*µg*/ml streptomycin sulfate, 110µg/ml pyruvic acid and 300*µg*/ml L-glutamine. C6 glioma cells, permanent transfected with the *δ* opioid receptor, required a DMEM medium, enriched with 10% heat inactivated fetal calf serum and the antibiotic solution as described above.

### Membrane preparation

The membranes were prepared as total particulate fractions. All cell lines were cultured to 90% confluency on 145 mm Petri dishes and treated with 5 mM sodium butyrate, 24 hours before collection. The culturing medium was removed and the cells were washed with ice cold phosphate buffered saline (PBS w/o Ca²⁺ and Mg²⁺), scraped from the plates in 50 mM Tris-HCl buffer, pH 7.4, and collected through centrifugation (10 minutes at 16,000 RPM at 4°C). The cell pellet was re-suspended in hypotonic 5 mM Tris-HCl buffer, pH 7.4, and re-homogenized with an Ultra Turrax homogenizer. The homogenate was centrifuged at 18000 RPM for 20 minutes at 4°C. The final pellet was re-suspended in 50 mM Tris-HCl buffer, pH 7.4 and stored in aliquots at -70°C.
A protein determination was performed using the Biorad protein assay (Bradford) using bovine serum albumine (BSA) as a standard *(Bradford, M.M.: A rapid and sensitive method for the quantification of microgram quantities of protein utilizing the principle of protein-dye binding. Analytical Biochem. 72*: *248-254*, *1976).*

### Radioligand binding

Preliminary radioligand binding experiments were carried out to reveal the optimal assay conditions for these opioid receptor subtypes in their corresponding mammalian cell membranes.
Competitive inhibition of [³H]DPDPE by the compounds was performed with a concentration of the radioligand of 2 nM (K_{d} = 1.7 nM) and various concentrations in singlet of the compounds, spanning at least 3 orders of magnitude around the pIC₅₀ value. For competition binding on the κ and µ receptor, [³H]U69593 (K_{d} = 0.4 nM) and [³H]DAMGO (K_{d} = 0.6 nM) were used respectively at a concentration of 1 nM. Membranes were thawed on ice and diluted in a 50 mM Tris-HCl buffer, pH 7.4. For the δ opioid receptor, this incubation buffer was supplemented with 2 mM MgCl₂, 1 mM EGTA and 0.1 % BSA. Non-specific binding was defined in the presence of 1 µM of naltrindole, spiradoline and dextromoramide for the δ, κ, and µ opioid receptor, respectively. An incubation of 1 hour at 25°C was found to be optimal for competition binding assays for all the three receptor subtypes. The assays were carried out in a final volume of 500 µl. The reaction was terminated by rapid filtration over an UniFilter^{™}-96, GF/B^{™} under reduced pressure using Filtermate 196 (Packard). The amount of bound radioactivity on the filter unit was determined after filter drying and scintillant addition (Microscint-O; Packard) by liquid scintillation counting.

### Signal transport binding

### 1) [³⁵S]GTPγS binding

Determination of [³⁵S]GTPγS binding to the G-proteins was carried out with a modified procedure of Lazareno *(Lazareno S.: Measurement of agonist-stimulated[³⁵S]GTPγS binding to cell membranes. Meth. Molec. Biol. 106, 231-243, 1999).*
In preliminary [³⁵S]GTPγS binding experiments, assay conditions were optimized which resulted in the choice of the following buffers: 20 mM Hepes with 100 mM NaCl, containing 3 µM GDP and 1 mM MgCl₂ for the µ opioid receptor CHO membranes, containing 10 µM GDP and 1 mM MgCl₂ for the δ opioid receptor C6 glioma cell membranes, and 10 µM GDP and 0.3 mM MgCl₂ for the κ opioid receptor CHO membranes . The assay mixtures contained 10 µg of membrane protein. An additional 10 µg/ml saponine was added to the diluted membranes as a detergent to maximize the [³⁵S]GTPγS penetration through the membranes.
For testing agonistic activity, 175 µl of diluted membranes was pre-incubated in the buffer described above together with 25 µl of buffer and 25 µl of varying concentrations of the compound in a total volume of 225 µl. For antagonistic activities, the 25 µl of the buffer addition was replaced with the reference agonist for stimulating the basal levels. For all three cell lines, a concentration of 300 nM of DPDPE, U69593 and morphine were used for their corresponding receptor subtypes. After a 20 minutes pre-incubation period at 37°C, 25 µl of [³⁵S]GTPγS was added to a final concentration of 0.25 nM and the assay mixtures were further incubated for 20 minutes at 37°C. Bound and free [³⁵S]GTPγS were separated by rapid filtration over an UniFilter^{™}-96, GF/B^{™} under reduced pressure using Filtermate 196 (Packard). The amount of bound radioactivity on the filter unit was determined after filter drying and scintillant addition (Microscint-O; Packard) by liquid scintillation counting. Basal [³⁵S]GTPγS binding was measured in absence of compounds. Stimulation by agonist was calculated as the percentage increase above basal levels. The sigmoid agonist concentration response curves for increases in [³⁵S]GTPγS binding and antagonist inhibition curves for inhibition of the reference agonist-stimulated [³⁵S]GTPγS binding were analyzed by non-linear regression using the GraphPad Prism program. Data were retrieved from independent experiments and the different concentration points were run in duplicates.

### 2) cAMP binding

### Cell culture

CHO cells were cultured in Dulbecco's modified Eagle's medium (DMEM)/ Nutrient mixture Ham's F12 (ratio 1:1) supplemented with 10% heat inactivated fetal calf serum, and an antibiotic solution containing 100 IU/ml penicillin G, 100 µg/ml streptomycin sulfate, 110 µg/ml pyruvic acid and 300 µg/ml L-glutamine. C6 glioma cells required a DMEM medium, enriched with 10% heat inactivated fetal calf serum and the antibiotic solution as described above.
The cells were grown in 175 cm² culture flasks at 37 °C in a 5% CO₂ environment. Two days before the experiment was run, the cells were seeded out in a 96 well plate at a density required for an approximately 90% cell confluency at the day of the experiment. Receptor expression in C6 glioma cells was induced with 5 mM sodium butyrate, 24 h before the assay.

### Adenylyl cyclase assay

After removal of the grow medium, the cells were washed twice with a controlled salt solution (CSS), pH 7.4 containing 25 mM Tris-HCl, 120 mM NaCl, 5.4 mM KCl, 0.8 mM MgCl₂, 1.8 mM CaCl₂, 15 mM glucose and 15 mg/l phenolred, with an addition of a phosphodiesterase inhibitor IBMX (3-isobutyl-1-methylxanthine) in a concentration of 1 mM and 0.1 % BSA. Basal cAMP levels were determined in CSS containing these additives and maximally stimulated cAMP levels were determined in the presence of 50 µM forskolin. In tests for agonistic activity, the compounds were incubated with the cells for 20 minutes at 37 °C. To test antagonistic activity, the cells were pre-incubated with different concentrations of the compound for 20 minutes at 37°C before the addition of the compound with 50 µM forskolin and 300 nM morphine (for the µ opioid receptor) and 10 nM DPDPE (for the δ opioid receptor). The reaction was stopped by addition of ice cold HClO₄ (1N) and stored at -20°C. The mixtures were neutralized after thawing with an equivalent amount of phosphate buffered KOH, left at 4°C during 30 minutes to allow the salts to precipitate and centrifuged at 2,000 RPM for 5 minutes at 4°C. For the quantitative determination of the cAMP levels, a 96-well Flashplate radioimmuno assay kit from NEN was used, according to the protocol of the supplier.

All compounds according to the invention showed a pICso value of at least 6 for the delta opioid receptor and a plC₅₀ value of 6 or less for either mu and kappa receptor.

The compounds listed in Table 7 showed a pIC₅₀ value of between 7 and 8 for the delta opioid receptor and a pIC₅₀ value of 6 or less for either mu and kappa receptor.

The compounds listed in Table 8 showed a pIC₅₀ value above 8 for the delta opioid receptor and a pIC₅₀ value of 6 or less for either mu and kappa receptor. The selectivity for the delta opioid receptor over the mu opioid receptor is as high as 600.

**Table 7 : pIC₅₀ values for the delta opioid receptor agonist test.**

| Comp. Nr. | pIC₅₀ | Comp. Nr. | pIC₅₀ |
|---|---|---|---|
| 43 | 7.9 | 22 | 7.3 |
| 17 | 7.9 | 87 | 7.3 |
| 30 | 7.9 | 45 | 7.3 |
| 105 | 7.9 | 51 | 7.3 |
| 78 | 7.9 | 4 | 7.3 |
| 101 | 7.8 | 55 | 7.3 |
| 28 | 7.8 | 71 | 7.3 |
| 11 | 7.8 | 99 | 7.3 |
| 29 | 7.8 | 34 | 7.2 |
| 67 | 7.8 | 72 | 7.2 |
| 7 | 7.7 | 81 | 7.2 |
| 9 | 7.7 | 64 | 7.2 |
| 52 | 7.7 | 18 | 7.2 |
| 103 | 7.7 | 42 | 7.2 |
| 26 | 7.7 | 10 | 7.2 |
| 27 | 7.7 | 33 | 7.1 |
| 15 | 7.6 | 37 | 7.1 |
| 69 | 7.6 | 80 | 7.1 |
| 50 | 7.6 | 90 | 7.1 |
| 32 | 7.6 | 56 | 7.1 |
| 93 | 7.5 | 47 | 7.1 |
| 65 | 7.5 | 43 | 7.1 |
| 84 | 7.5 | 48 | 7.1 |
| 66 | 7.5 | 79 | 7.0 |
| 75 | 7.4 | 111 | 7.0 |
| 13 | 7.4 | 117 | 7.0 |
| 76 | 7.4 | 68 | 7.0 |
| 96 | 7.4 | 95 | 7.0 |
| 94 | 7.4 | 92 | 7.0 |
| 70 | 7.4 | 49 | 7.0 |
| 36 | 7.3 | 74 | 7.0 |

**Table 8 : Results for the agonist receptor binding (pIC₅₀) and signal transport binding (pIC₅₀) testing. n.d. : not determined**

| Comp. Nr. | Formula | Agonist receptor binding | | | Signal transport binding | | |
|---|---|---|---|---|---|---|---|
| | | (pIC₅₀) | | | (pIC₅₀) | | |
| | | delta | mu | kappa | delta agonism GTPγS | delta agonism cAMP | delta antag. |
| 3 | 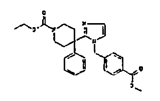 | 8.8 | <6 | n.d. | 7.3 | 7.8 | <5 |
| 38 | 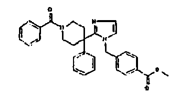 | 8.7 | <6 | n.d. | n.d. | 8.3 | n.d. |
| 20 | 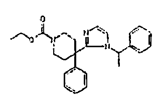 | 8.6 | <6 | n.d. | 7 | 7.5 | <5 |
| 102 | 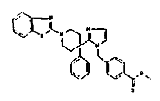 | 8.5 | <6 | n.d. | n.d. | n.d. | n.d. |
| 25 | 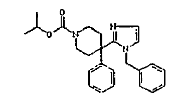 | 8.4 | <6 | n.d. | 6.9 | 8.0 | <5 |
| 2 | 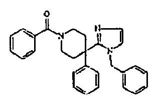 | 8.3 | <6 | n.d. | 6.8 | 7.8 | <5 |
| 41 | 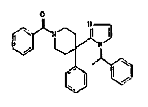 | 8.3 | <6 | n.d. | n.d. | n.d. | n.d. |
| 98 | 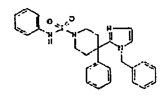 | 8.2 | 5.6 | 5.8 | 6.1 | n.d. | <5 |
| 19 | 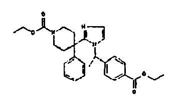 | 8.2 | <6 | n.d. | 6.5 | n.d. | <5 |
| 24 | 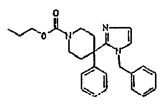 | 8.2 | <6 | n.d. | 6.9 | 8.2 | <5 |
| 1 | 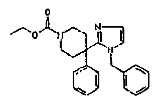 | 8.1 | <5 | 6.3 | 7.1 | 7.6 | <5 |
| 31 | 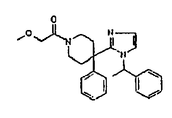 | 8.1 | <6 | n.d. | n.d. | 8.1 | n.d. |
| 12 | 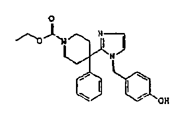 | 8.0 | <6 | n.d. | 7 | 7.9 | <5 |

Antidepressant- and anxiolytic like properties of the compounds were examined in mouse tail suspension test (TS), mouse forced swim test (FS) and mouse neonatal ultrasonic vocalization test (USV).

### Mouse tail suspension

This test has been validated as test for antidepressant-like activity (Porsolt et al., *Psychopharmacology* 1986, 89, S28; Steru et al., *Psychopharmacology* 1985, 85, 367). Antidepressants have been shown to decrease the duration of immobility.

Adult male NMRI mice (body weight 20 - 22 g; Iffa Credo, Brussels, Belgium) were used. Animals were housed individually in IVC racks and maintained under a 12:12 h light/dark cycle (lights on at 6:00 h), with food and water ad libitum.

Tail suspension was measured in two test set ups, each consisting of three test chambers (15 x 15 x 19 cm high) made of white plastic walls and black plastic floor (Biosep, France). Each test chamber was equipped with a metal hook at its ceiling, which allowed to hang an animal by its tail, using adhesive tape. Animals were unable to see each other. Each hook was connected to a computerised strain gauge that was adjusted to detect all movements of the animals (Tail Suspension Test software, Biosep). Each test set up was surrounded by a sound attenuating, ventilated chamber.

Thirty minutes after administration of the test compound (i.p., 10 ml/kg), animals were suspended by their tails. Recording started 30 s afterwards. Movements of the mice were measured over 6 min. Immobility was defined as total time not moving. Power of movement was measured as the relative amplitude of the movements made by the animals. Six animals were tested in parallel.

Data were analyzed using the non-parametric, unadjusted Wilcoxon-Mann-Whitney rank sum test. Each dose was compared with vehicle.

### Mouse forced swim

This test has been validated as test for antidepressant-like activity (Borsini and Meli, *Psychopharmacology* 1988, 94, 147). Antidepressant treatments have been shown to decrease the duration of immobility.

Adult male C57BL/6 mice (body weight 22 - 24g; Charles River, Sulzfeld, Germany) were were housed individually in IVC racks and maintained under a 12:12 h light/dark cycle (lights on at 6:00 h), with food and water ad libitum.

A clear glass beaker (10 cm in diameter, 25 cm high) was filled to a depth of 10 cm with water (25 °C). Mice were placed into the water for 6 min. Immobility during the first and the last 3 min were scored automatically using the Videotrack video tracking system (Viewpoint, France), which allows detection of individual pixel movements. Four animals were tested in parallel. Treatment was given 30 min prior to test (s.c., 10 ml/kg). The water was replaced after each animal.

Data were analyzed using the non-parametric, unadjusted Wilcoxon-Mann-Whitney rank sum test. Each dose was compared with vehicle.

### Mouse neonatal ultrasonic vocalization

Anxiolytic compounds typically decrease the vocalizations in rat pups separated from their mother (Olivier et al., *Eur. J. Pharmacol.* 1998, 358, 117). More recently, this test has also been established in mice (Dirks et al., *Pharmacol. Biochem. Behav.* 2002, 72, 993).

Seventeen days pregnant C57BL/6 mice were obtained from Charles River (Sulzfeld, Germany). They were housed and maintained at a constant temperature of 22 °C, under a 12:12 h reversed light/dark cycle (lights off at 6:00 h), with food and water ad libitum. Two times per day, cages were checked for newborn litters. Litter size was 5 - 8 pups/litter.

Ultrasonic vocalizations (USVs) were recorded in a sound attenuating, ventilated chamber at room temperature, detected at 60 kHz (range 50 - 70 kHz) by a bat recorder in combination with the UltraVox system (Noldus, The Netherlands). No filter was used in order to be able to record pup clicks.

Distress calls of neonatal mice were measured in neonatal C57BL/6 mice at postnatal day 3 during the early dark phase (between 8:00 and 12:00). Pups were weighed (body weight 2 - 3 g), followed by subcutaneous injection (10 ml/kg), and placed back into the nest. After 30 min, they were separated from the mother and placed into a beaker underneath the bat detector. The number of calls and the duration of calls were recorded over a period of 5 min.

Data were analyzed using the non-parametric, unadjusted Wilcoxon-Mann-Whitney rank sum test. Each dose was compared with vehicle.

Antidepressant- and anxiolytic-like activity was observed with 2 compounds tested (Table 9).

**Table 9: Effects of delta opioid agonists on animal models of depression and anxiety (LAD = Lowest Active Dose tested; n.d. = not determined)**

| Compound | Tail suspension (LAD) | Forced swim (LAD,) | Ultrasonic vocalization (LAD) |
|---|---|---|---|
| 1 | 30 mg/kg s.c. | 30 mg/kg s.c. | 10 mg/kg s.c. |
| 2 | 10 mg/kg s.c. | n.d. | 1 mg/kg s.c. |
| 20 | 30 mg/kg s.c. | n.d. | 30 mg/kg s.c. |

## Claims

1. Use of a compound according to Formula (I) 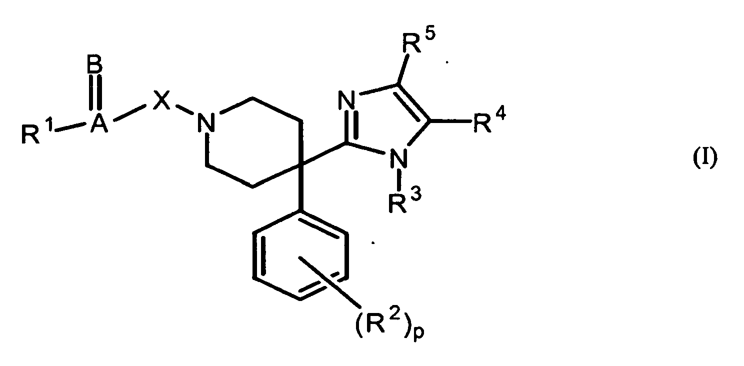 the pharmaceutically acceptable acid or base addition salts thereof, the stereochemically isomeric forms thereof, the tautomeric forms thereof and the *N*-oxide forms thereof, for the manufacture of a medicament for the prevention and/or treatment of central nervous system disorders, selected from the group consisting of mood disorders, depressive disorders, anxiety disorders, stress-related disorders associated with depression and/or anxiety and eating disorders, or a combination thereof, wherein :
A=B is C=O, C=N-R⁶ wherein R⁶ is hydrogen or cyano, C=S, S=O, SO₂ and C=CR⁷R⁸ wherein R⁷ and R⁸ each independently are hydrogen, nitro or alkyl ;
X is a covalent bond, -CH₂- or CH₂CH₂- ;
R¹ is hydrogen, hydroxy, alkyloxy, alkylcarbonyloxy, Ar-oxy, Het-oxy, Ar-carbonyloxy, Het-carbonyloxy, Ar-alkyloxy, Het-alkyloxy, alkyl, polyhaloalkyl, alkyloxyalkyl, Ar-alkyl, Het-alkyl, Ar, Het, thio, alkylthio, Ar-thio, Het-thio or NR⁹R¹⁰ wherein R⁹ and R¹⁰ each independently are hydrogen, alkyl, Ar, Ar-alkyl, Het, Het-alkyl, Ar-carbonyl, alkylcarbonyl, Het-carbonyl or alkyloxycarbonylalkyl ;
or A=B and R¹ together form an optionally substituted semi-aromatic or aromatic carbocyclic or heterocyclic radical Het² or Het³;
R² is hydroxy, alkyloxy, alkylcarbonyloxy, phenyloxy, phenylcarbonyloxy, halo, cyano, alkyl, polyhaloalkyl, alkyloxyalkyl, formyl, carboxy, alkylcarbonyl, alkyloxycarbonyl, aminocarbonyl, mono- or dialkylaminocarbonyl, phenyl, nitro, amino, mono- or dialkyl-amino, thio or alkylthio ;
R³ is alkyl, Ar, Ar-alkyl, Ar-alkenyl, Ar-carbonyl, Het, Het-alkyl, Het-alkenyl or Het-carbonyl ;
R⁴, R⁵ each independently is hydrogen, alkyl, carboxy; aminocarbonyl, alkyloxycarbonyl, halo or hydroxyalkyl ;
p is an integer equal to zero, 1, 2 or 3 ;
alkyl is a straight or branched saturated hydrocarbon radical having from 1 to 6 carbon atoms ; or is a cyclic saturated hydrocarbon (cycloalkyl) radical having from 3 to 7 carbon atoms ; or is a cyclic saturated hydrocarbon radical having from 3 to 7 carbon atoms attached to a straight or branched saturated hydrocarbon radical having from 1 to 6 carbon atoms; wherein each carbon atom may be optionally substituted with amino, nitro, thio, hydroxy, oxo, cyano, formyl or carboxy ;
alkenyl is an alkyl radical having one or more double bonds ;
Ar is a homocycle selected from the group of phenyl and naphthyl, each optionally substituted with one or more substituents, each substituent independently selected from the group of hydroxy, alkyloxy, alkylcarbonyloxy, phenyloxy, phenylcarbonyloxy, polyhaloalkyloxy, halo, cyano, alkyl, polyhaloalkyl, alkyloxyalkyl, formyl, haloformyl, carboxy, alkylcarbonyl, alkyloxycarbonyl, aminocarbonyl, mono- or dialkylaminocarbonyl, phenylalkyl, phenyl, nitro, amino, mono- or dialkyl-amino, thio, alkylthio or SO₂-CH₃;
halo is a substituent selected from the group of fluoro, chloro, bromo and iodo ;
polyhaloalkyl is a straight or branched saturated hydrocarbon radical having from 1 to 6 carbon atoms or a cyclic saturated hydrocarbon radical having from 3 to 7carbon atoms, wherein one or more carbon atoms is substituted with one or more halo-atoms ;
Het is a heterocyclic radical selected from the group of Het¹, Het² and Het³ ; wherein each heterocyclic radical Het¹, Het² and Het³ may optionally be substituted on a carbon and/or an heteroatom with halo, hydroxy, alkyloxy, alkyl, Ar, Ar-alkyl or pyridinyl.
Het¹ is an aliphatic monocyclic heterocyclic radical selected from the group of pyrrolidinyl, dioxolyl, imidazolidinyl, pyrrazolidinyl, piperidinyl, dioxyl, morpholinyl, dithianyl, thiomorpholinyl, piperazinyl and tetrahydrofuranyl ;
Het² is a semi-aromatic monocyclic heterocyclic radical selected from the group of *2H*-pyrrolyl, pyrrolinyl, imidazolinyl and pyrrazolinyl ;
Het³ is an aromatic monocyclic heterocyclic radical selected from the group of pyrrolyl, pyrazolyl, imidazolyl, furanyl, thienyl, oxazolyl, isoxazolyl, thiazolyl, isothiazolyl, pyridinyl, pyrimidinyl, pyrazinyl, pyridazinyl and triazinyl; or an aromatic bicyclic heterocyclic radical selected from the group of quinolinyl, quinoxalinyl, indolyl, benzimidazolyl, benzoxazolyl, benzisoxazolyl, benzothiazolyl, benzisothiazolyl, benzofuranyl and benzothienyl.

2. Use according to claim 1, **characterized in that** R¹ is selected from the group of alkyloxy, Ar-alkyloxy, alkyl, polyhaloalkyl, alkyloxyalkyl, Ar-alkyl, Het-alkyl, Ar, piperazinyl, pyrrolyl, thiazolyl, pyrrolidinyl and NR⁹R¹⁰ wherein R⁹ and R¹⁰ each independently are hydrogen, alkyl, Ar, Ar-alkyl, pyridinyl or alkyloxycarbonylalkyl.

3. Use according to claim 1, **characterized in that** A=B and R¹ together form a radical selected from the group of Het² and Het³.

4. Use according to claim 3, **characterized in that** A=B and R¹ together form a radical selected from the group of benzoxazolyl, thiazolyl, benzothiazolyl, benzimidazolyl and pyrimidinyl.

5. Use according to any one of claims 1-4, **characterized in that** X is a covalent bond.

6. Use according to any one of claims 1-5, **characterized in that** R² is alkyloxy or halo.

7. Use according to any one of claims 1-6, **characterized in that** R³ is selected from the group of phenylalkyl and naphthyl, each independently substituted with at least one substituent selected from the group of halo, alkyloxycarbonyl, hydroxy, alkyloxy and dialkylaminocarbonyl.

8. Use according to claim 1, in which A=B is C=O or SO₂, R¹ is alkyloxy, alkyloxyalkyl, Ar or NR⁹R¹⁰, wherein R⁹ and R¹⁰ each independently are hydrogen or Ar ; or A=B and R¹ together form a benzoxazolyl radical ; p is zero, R³ is benzyl optionally substituted with hydroxy or alkyloxycarbonyl and R⁴ and R⁵ each are hydrogen.

9. Use according to claim 1, wherein the compound is selected from the group of
- 4-[[2-(1-benzoyl-4-phenyl-4-piperidinyl)-1*H*-imidazol-1-yl]methyl]-methylbenzoate ;
- 1-ethoxycarbonyl-4-phenyl-4-[1-(1-phenylethyl)-1*H*-imidazol-2-yl]-piperidine ;
- 4-[[2-[1-(2-benzoxazolyl)-4-phenyl-4-piperidinyl]-1*H*-imidazol-1-yl]methyl]-methylbenzoate ;
- 1-benzoyl-4-phenyl-4-[1-(phenylmethyl)-1*H*-imidazol-2-yl]-piperidine ;
- 1-benzoyl-4-phenyl-4-[1-(1-phenylethyl)-1*H*-imidazol-2-yl]-piperidine ;
- *N*,4-diphenyl-4-[1-(phenylmethyl)-1*H*-imidazol-2-yl]-1-piperidine-sulfonamide ;
- 1-ethoxycarbonyl-4-phenyl-4-[1-(phenylmethyl)-1*H* imidazol-2-yl]-piperidine ;
- 1-(methoxyacetyl)-4-phenyl-4-[1-(1-phenylethyl)-1*H*-imidazol-2-yl]-piperidine ;
- [4-(1-Benzyl-1*H*-imidazol-2-yl)-4-phenyl-piperidin-1-yl]-(3,5-dimethylphenyl)-methanone ;
- 4-{2-[1-(2-Methoxy-acetyl)-4-phenyl-piperidin-4-yl]-imidazol-1-ylmethyl}-methylbenzoate ;
- 4-(1-Benzyl-1*H*-imidazol-2-yl)-4-phenyl-1-thiazol-2-yl-piperidine
- 2-{4-Phenyl-4-[1-(1-phenyl-ethyl)-1*H*-imidazol-2-yl]-piperidin-1-yl}-benzo-oxazole ;
- 1-[4-(1-Benzyl-1*H*-imidazol-2-yl)-4-phenyl-piperidin-1-yl]-2-methoxy-ethanone ; and
- 2-[4-(1-Benzyl-1*H* imidazol-2-yl)-4-phenyl-piperidin-1-yl]-pyrimidine.

10. Use according to any one of claims 1-9, **characterized in that** the central nervous system disorder is a depressive and/or anxiety disorder.

11. Use according to any one of claims 1-10, **characterized in that** the compounds according to Formula (I), the pharmaceutically acceptable acid or base addition salts thereof, the stereochemically isomeric forms thereof, the tautomeric forms thereof and the *N*-oxide forms thereof are co-administered with other antidepressant, anti-anxiety and/or antipsychotic agents.

12. Use according to claim 11, in that the compounds according to Formula (I), the pharmaceutically acceptable acid or base addition salts thereof, the stereochemically isomeric forms thereof, the tautomeric forms thereof and the *N*-oxide forms thereof and the other agents may be present as a combined preparation for simultaneous, separate or sequential use.

## Patentansprüche

1. Verwendung einer Verbindung der Formel (I) 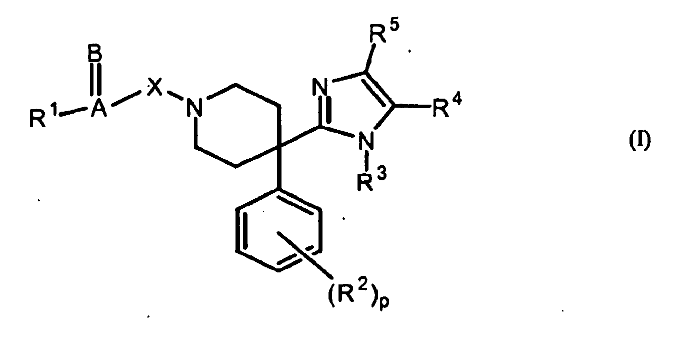 der pharmazeutisch annehmbaren Säure- oder Basenadditionssalze davon, der stereochemisch isomeren Formen davon, der tautomeren Formen davon und der *N*-Oxid-Formen davon, zur Herstellung eines Medikaments zur Vorbeugung und/oder Behandlung von Störungen des zentralen Nervensystems, ausgewählt aus der Gruppe, bestehend aus Stimmungsstörungen, depressiven Störungen, Angststörungen, mit Stress zusammenhängenden Störungen in Verbindung mit Depression und/oder Angst und Essstörungen oder einer Kombination davon, wobei:
A=B für C=O, C=N-R⁶, wobei R⁶ Wasserstoff oder Cyano ist, C=S, S=O, SO₂ und C=CR⁷R⁸ steht, wobei R⁷ und R⁸ jeweils unabhängig Wasserstoff, Nitro oder Alkyl sind;
X eine kovalente Bindung, -CH₂- oder CH₂CH₂- ist;
R¹ Wasserstoff, Hydroxy, Alkyloxy, Alkylcarbonyloxy, Ar-oxy, Het-oxy, Ar-carbonyloxy, Het-carbonyloxy, Ar-alkyloxy, Het-alkyloxy, Alkyl, Polyhalogenalkyl, Alkyloxyalkyl, Ar-alkyl, Het-alkyl, Ar, Het, Thio, Alkylthio, Ar-thio, Het-thio oder NR⁹R¹⁰ ist, wobei R⁹ und R¹⁰ jeweils unabhängig Wasserstoff, Alkyl, Ar, Ar-alkyl, Het, Het-alkyl, Ar-carbonyl, Alkylcarbonyl, Het-carbonyl oder Alkyloxycarbonylalkyl sind;
oder A=B und R¹ zusammen einen gegebenenfalls substituierten semiaromatischen oder aromatischen carbocyclischen oder heterocyclischen Rest Het² oder Het³ bilden;
R² Hydroxy, Alkyloxy, Alkylcarbonyloxy, Phenyloxy, Phenylcarbonyloxy, Halogen, Cyano, Alkyl, Polyhalogenalkyl, Alkyloxyalkyl, Formyl, Carboxy, Alkylcarbonyl, Alkyloxycarbonyl, Aminocarbonyl, Mono- oder Dialkylaminocarbonyl, Phenyl, Nitro, Amino, Mono- oder Dialkylamino, Thio oder Alkylthio ist;
R³ Alkyl, Ar, Ar-alkyl, Ar-alkenyl, Ar-carbonyl, Het, Het-alkyl, Het-alkenyl oder Het-carbonyl ist;
R⁴, R⁵ jeweils unabhängig Wasserstoff, Alkyl, Carboxy; Aminocarbonyl, Alkyloxycarbonyl, Halogen oder Hydroxyalkyl ist;
p eine ganze Zahl gleich Null, 1, 2 oder 3 ist;
Alkyl ein gerader oder verzweigter gesättigter Kohlenwasserstoffrest mit 1 bis 6 Kohlenstoffatomen ist; oder ein cyclischer gesättigter Kohlenwasserstoff- (Cycloalkyl-) rest mit 3 bis 7 Kohlenstoffatomen ist; oder ein cyclischer gesättigter Kohlenwasserstoffrest mit 3 bis 7 Kohlenstoffatomen, der an einen geraden oder verzweigten gesättigten Kohlenwasserstoffrest mit 1 bis 6 Kohlenstoffatomen gebunden ist, ist, wobei jedes Kohlenstoffatom gegebenenfalls mit Amino, Nitro, Thio, Hydroxy, Oxo, Cyano, Formyl oder Carboxy substituiert ist;
Alkenyl ein Alkylrest mit einer oder mehreren Doppelbindungen ist;
Ar ein Homocyclus ist, der aus der Gruppe mit Phenyl und Naphthyl ausgewählt ist, die jeweils gegebenenfalls mit einem oder mehreren Substituenten substituiert sind, wobei jeder Substituent unabhängig aus der Gruppe mit Hydroxy, Alkyloxy, Alkylcarbonyloxy, Phenyloxy, Phenylcarbonyloxy, Polyhalogenalkyloxy, Halogen, Cyano, Alkyl, Polyhalogenalkyl, Alkyloxyalkyl, Formyl, Halogenformyl, Carboxy, Alkylcarbonyl, Alkyloxycarbonyl, Aminocarbonyl, Mono- oder Dialkylaminocarbonyl, Phenylalkyl, Phenyl, Nitro, Amino, Mono- oder Dialkylamino, Thio, Alkylthio oder SO₂-CH₃ ausgewählt ist;
Halogen ein Substituent ist, der aus der Gruppe mit Fluor, Chlor, Brom und Iod ausgewählt ist; Polyhalogenalkyl ein gerader oder verzweigter gesättigter Kohlenwasserstoffrest mit 1 bis 6 Kohlenstoffatomen oder ein cyclischer gesättigter Kohlenwasserstoffrest mit 3 bis 7 Kohlenstoffatomen ist, wobei ein oder mehrere Kohlenstoffatom(e) mit einem oder mehreren Halogenatomen substituiert ist/sind;
Het ein heterocyclischer Rest ist, der aus der Gruppe mit Het¹, Het² und Het³ ausgewählt ist, wobei jeder heterocyclische Rest Het¹, Het² und Het³ gegebenenfalls an einem Kohlenstoff- und/oder einem Heteroatom mit Halogen, Hydroxy, Alkyloxy, Alkyl, Ar, Ar-alkyl oder Pyridinyl substituiert sein kann;
Het¹ ein aliphatischer, monocyclischer, heterocyclischer Rest ist, der aus der Gruppe mit Pyrrolidinyl, Dioxolyl, Imidazolidinyl, Pyrrazolidinyl, Piperidinyl, Dioxyl, Morpholinyl, Dithianyl, Thiomorpholinyl, Piperazinyl und Tetrahydrofuranyl ausgewählt ist;
Het² ein semiaromatischer, monocyclischer, heterocyclischer Rest ist, der aus der Gruppe mit 2H-Pyrrolyl, Pyrrolinyl, Imidazolinyl und Pyrrazolinyl ausgewählt ist;
Het³ ein aromatischer, monocyclischer, heterocyclischer Rest ist, der aus der Gruppe mit Pyrrolyl, Pyrazolyl, Imidazolyl, Furanyl, Thienyl, Oxazolyl, Isoxazolyl, Thiazolyl, Isothiazolyl, Pyridinyl, Pyrimidinyl, Pyrazinyl, Pyridazinyl und Triazinyl ausgewählt ist; oder ein aromatischer bicyclischer heterocyclischer Rest ist, der aus der Gruppe mit Chinolinyl, Chinoxalinyl, Indolyl, Benzimidazolyl, Benzoxazolyl, Benzisoxazolyl, Benzothiazolyl, Benzisothiazolyl, Benzofuranyl und Benzothienyl ausgewählt ist.

2. Verwendung nach Anspruch 1, **dadurch gekennzeichnet, dass** R¹ aus der Gruppe mit Alkyloxy, Ar-alkyloxy, Alkyl, Polyhalogenalkyl, Alkyloxyalkyl, Ar-alkyl, Het-alkyl, Ar, Piperazinyl, Pyrrolyl, Thiazolyl, Pyrrolidinyl und NR⁹R¹⁰ ausgewählt ist, wobei R⁹ und R¹⁰ jeweils unabhängig Wasserstoff, Alkyl, Ar, Ar-alkyl, Pyridinyl oder Alkyloxycarbonylalkyl sind.

3. Verwendung nach Anspruch 1, **dadurch gekennzeichnet, dass** A=B und R¹ zusammen einen Rest bilden, der aus der Gruppe mit Het² und Het³ ausgewählt ist.

4. Verwendung nach Anspruch 3, **dadurch gekennzeichnet, dass** A=B und R¹ zusammen einen Rest bilden, der aus der Gruppe mit Benzoxazolyl, Thiazolyl, Benzothiazolyl, Benzimidazolyl und Pyrimidinyl ausgewählt ist.

5. Verwendung nach einem der Ansprüche 1-4, **dadurch gekennzeichnet, dass** X eine kovalente Bindung ist.

6. Verwendung nach einem der Ansprüche 1-5, **dadurch gekennzeichnet, dass** R² Alkyloxy oder Halogen ist.

7. Verwendung nach einem der Ansprüche 1-6, **dadurch gekennzeichnet, dass** R³ aus der Gruppe mit Phenylalkyl und Naphthyl ausgewählt ist, die jeweils unabhängig mit mindestens einem Substituenten substituiert sind, der aus der Gruppe mit Halogen, Alkyloxycarbonyl, Hydroxy, Alkyloxy und Dialkylaminocarbonyl ausgewählt ist.

8. Verwendung nach Anspruch 1, wobei A=B für C=O oder SO₂ steht, R¹ Alkyloxy, Alkyloxyalkyl, Ar oder NR⁹R¹⁰ ist, wobei R⁹ und R¹⁰ jeweils unabhängig Wasserstoff oder Ar sind; oder A=B und R¹ zusammen einen Benzoxazolylrest bilden; p Null ist, R³ gegebenenfalls mit Hydroxy oder Alkyloxycarbonyl substituiertes Benzyl ist und R⁴ und R⁵ jeweils Wasserstoff sind.

9. Verwendung nach Anspruch 1, wobei die Verbindung aus Gruppe mit
- 4-[[2-(1-Benzoyl-4-phenyl-4-piperidinyl)-1*H-*imidazol-1-yl]methyl]-methylbenzoat;
- 1-Ethoxycarbonyl-4-phenyl-4-[1-(1-phenylethyl)-1*H*-imidazol-2-yl]piperidin;
- 4-[[2-[1-(2-Benzoxazolyl)-4-phenyl-4-piperidinyl]-1*H*-imidazol-1-yl]methyl]-methylbenzoat;
- 1-Benzoyl-4-phenyl-4-[1-(phenylmethyl)-1*H-*imidazol-2-yl]piperidin;
- 1-Benzoyl-4-phenyl-4-[1-(1-phenylethyl)-1*H-*imidazol-2-yl]piperidin;
- *N*,4-Diphenyl-4-[1-(phenylmethyl)-1*H*-imidazol-2-yl]-1-piperidinsulfonamid;
- 1-Ethoxycarbonyl-4-phenyl-4-[1-(phenylmethyl)-1*H*-imidazol-2-yl]piperidin;
- 1-(Methoxyacetyl)-4-phenyl-4-[1-(1-phenylethyl)-1*H*-imidazol-2-yl]piperidin;
- [4-(1-Benzyl-1*H*-imidazol-2-yl)-4-phenylpiperidin-1-yl]-(3,5-dimethylphenyl)-methanon;
- 4-{2-[1-(2-Methoxyacetyl)-4-phenylpiperidin-4-yll-imidazol-1-ylmethyl}-methylbenzoat;
- 4-(1-Benzyl-1*H*-imidazol-2-yl)-4-phenyl-1-thiazol-2-yl-piperidin;
- 2-{4-Phenyl-4-[1-(1-phenylethyl)-1*H*-imidazol-2-yl]-piperidin-1-yl)-benzoxazol;
- 1-[4-(1-Benzyl-1*H*-imidazol-2-yl)-4-phenylpiperidin-1-yl]-2-methoxyethanon; und
- 2-[4-(1-Benzyl-1*H*-imidazol-2-yl)-4-phenylpiperidin-1-yl]-pyrimidin.

10. Verwendung nach einem der Ansprüche 1-9, **dadurch gekennzeichnet, dass** die Störung des zentralen Nervensystems eine depressive und/oder eine Angststörung ist.

11. Verwendung nach einem der Ansprüche 1-10, **dadurch gekennzeichnet, dass** die Verbindungen der Formel (I), die pharmazeutisch annehmbaren Säure- oder Basenadditionssalze davon, die stereochemisch isomeren Formen davon, die tautomeren Formen davon und die *N*-Oxid-Formen davon zusammen mit anderen Antidepressiva, Anxiolytika und/oder Antipsychotika verabreicht werden.

12. Verwendung nach Anspruch 11, **dadurch gekennzeichnet, dass** die Verbindungen der Formel (I), die pharmazeutisch annehmbaren Säure- oder Basenadditionssalze davon, die stereochemisch isomeren Formen davon, die tautomeren Formen davon und die *N*-Oxid-Formen davon und die anderen Mittel als Kombinationszubereitung zur gleichzeitigen, getrennten oder aufeinander folgenden Verwendung vorliegen können.

## Revendications

1. Utilisation d'un composé selon la formule (I) 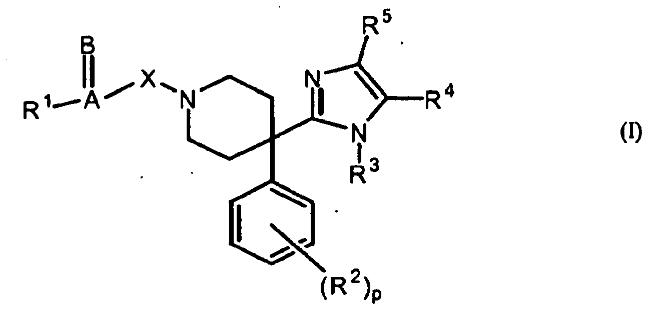 des sels d'addition d'acide ou de base pharmaceutiquement acceptables de celui-ci, des formes stéréochimiquement isomères de celui-ci, des formes tautomères de celui-ci et des formes N-oxydes de celui-ci, pour la préparation d'un médicament destiné à la prévention et/ou au traitement de troubles du système nerveux central, choisis dans le groupe constitué par les troubles de l'humeur, les troubles dépressifs, les troubles d'anxiété, les troubles relatifs au stress, associés à une dépression et/ou une anxiété et les troubles de l'appétit ou une combinaison de ceux-ci, où :
A=B représente C=O, C=N-R⁶ où R⁶ représente hydrogène ou cyano, C=S, S=O, SO₂ et C=CR⁷R⁸ où R⁷ et R⁸ représentent, chacun indépendamment, hydrogène, nitro ou alkyle ;
X est une liaison covalente, -CH₂- ou CH₂CH₂- ;
R¹ représente hydrogène, hydroxy, alkyloxy, alkylcarbonyloxy, Ar-oxy, Hét-oxy, Ar-carbonyloxy, Hét-carbonyloxy, Ar-alkyloxy, Hét-alkyloxy, alkyle, polyhalogénoalkyle, alkyloxyalkyle, Ar-alkyle, Hét-alkyle, Ar, Hét, thio, alkylthio, Ar-thio, Hét-thio ou NR⁹R¹⁰ où R⁹ et R¹⁰ représentent, chacun indépendamment, hydrogène, alkyle, Ar, Ar-alkyle, Hét, Hét-alkyle, Ar-carbonyle, alkylcarbonyle, Hét-carbonyle ou alkyloxycarbonylalkyle ; ou A=B et R¹ ensemble forment un radical carbocyclique ou hétérocyclique, semi-aromatique ou aromatique, éventuellement substitué Hét² ou Hét³ ;
R² représente hydroxy, alkyloxy, alkylcarbonyloxy, phényloxy, phénylcarbonyloxy, halogéno, cyano, alkyle, polyhalogénoalkyle, alkyloxyalkyle, formyle, carboxy, alkylcarbonyle, alkyloxycarbonyle, aminocarbonyle, monoalkylaminocarbonyle ou dialkylaminocarbonyle, phényle, nitro, amino, monoalkylamino ou dialkylamino, thio ou alkylthio ;
R³ représente alkyle, Ar, Ar-alkyle, Ar-alcényle, Ar-carbonyle, Hét, Hét-alkyle, Hét-alcényle ou Hét-carbonyle ;
R⁴, R⁵représentent, chacun indépendamment, hydrogène, alkyle, carboxy ; aminocarbonyle, alkyloxycarbonyle, halogéno ou hydroxyalkyle ;
p est un entier égal à zéro, 1, 2 ou 3 ;
alkyle représente un radical hydrocarboné saturé linéaire ou ramifié comprenant 1 à 6 atomes de carbone ; ou représente un radical hydrocarboné saturé cyclique (cycloalkyle) comprenant 3 à 7 atomes de carbone ; ou représente un radical hydrocarboné saturé cyclique comprenant 3 à 7 atomes de carbone attaché à un radical hydrocarboné saturé linéaire ou ramifié comprenant 1 à 6 atomes de carbone ; où chaque atome de carbone peut éventuellement être substitué par amino, nitro, thio, hydroxy, oxo, cyano, formyle ou carboxy ;
alcényle représente un radical alkyle présentant une ou plusieurs doubles liaisons ;
Ar est un homocycle choisi dans le groupe formé par phényle et naphtyle, à chaque fois éventuellement substitué par un ou plusieurs substituants, chaque substituant étant choisi indépendamment dans le groupe formé par hydroxy, alkyloxy, alkylcarbonyloxy, phényloxy, phénylcarbonyloxy, polyhalogénoalkyloxy, halogéno, cyano, alkyle, polyhalogénoalkyle, alkyloxyalkyle, formyle, halogénoformyle, carboxy, alkylcarbonyle, alkyloxycarbonyle, aminocarbonyle, monoalkylaminocarbonyle ou dialkylaminocarbonyle, phénylalkyle, phényle, nitro, amino, monoalkylamino ou dialkylamino, thio, alkylthio ou SO₂-CH₃ ;
halogéno est un substituant choisi dans le groupe formé par fluoro, chloro, bromo et iodo ;
polyhalogénoalkyle représente un radical hydrocarboné saturé linéaire ou ramifié, comprenant 1 à 6 atomes de carbone ou un radical hydrocarboné saturé cyclique comprenant 3 à 7 atomes de carbone, où un ou plusieurs atomes de carbone sont substitués par un ou plusieurs atomes d'halogène ;
Hét est un radical hétérocyclique choisi dans le groupe formé par Hét¹, Hét² et Hét³ ; où chaque radical hétérocyclique Hét¹, Hét² et Hét³ peut éventuellement être substitué sur un atome de carbone et/ou un hétéroatome par halogène, hydroxy, alkyloxy, alkyle, Ar, Ar-alkyle ou pyridinyle,
Hét¹ représente un radical hétérocyclique monocyclique aliphatique choisi dans le groupe formé par pyrrolidinyle, dioxolyle, imidazolidinyle, pyrrazolidinyle, pipéridinyle, dioxyle, morpholinyle, dithianyle, thiomorpholinyle, pipérazinyle et tétrahydrofurannyle ;
Hét² est un radical hétérocyclique monocyclique, semi-aromatique choisi dans le groupe formé par 2H-pyrrolyle, pyrrolinyle, imidazolinyle et pyrrazolinyle ;
Hét³ est un radical hétérocyclique monocyclique aromatique choisi dans le groupe formé par pyrrolyle, pyrazolyle, imidazolyle, furannyle, thiényle, oxazolyle, isoxazolyle, thiazolyle, isothiazolyle, pyridinyle, pyrimidinyle, pyrazinyle, pyridazinyle et triazinyle ; ou un radical hétérocyclique bicyclique aromatique choisi dans le groupe formé par quinoléinyle, quinoxalinyle, indolyle, benzimidazolyle, benzoxazolyle, benzisoxazolyle, benzothiazolyle, benzisothiazolyle, benzofurannyle et benzothiényle.

2. Utilisation selon la revendication 1, **caractérisée en ce que** R¹ est choisi dans le groupe formé par alkyloxy, Ar-alkyloxy, alkyle, polyhalogénoalkyle, alkyloxyalkyle, Ar-alkyle, Hét-alkyle, Ar, pipérazinyle, pyrrolyle, thiazolyle, pyrrolidinyle et NR⁹R¹⁰ où R⁹ et R¹⁰ représentent, chacun indépendamment, hydrogène, alkyle, Ar, Ar-alkyle, pyridinyle ou alkyloxycarbonylalkyle.

3. Utilisation selon la revendication 1, **caractérisée en ce que** A=B et R¹ ensemble forment un radical choisi dans le groupe formé par Hét² et Hét³.

4. Utilisation selon la revendication 3, **caractérisée en ce que** A=B et R¹ ensemble forment un radical choisi dans le groupe formé par benzoxazolyle, thiazolyle, benzothiazolyle, benzimidazolyle et pyrimidinyle.

5. Utilisation selon l'une quelconque des revendications 1-4, **caractérisée en ce que** X est une liaison covalente.

6. Utilisation selon l'une quelconque des revendications 1-5, **caractérisée en ce que** R² représente alkyloxy ou halogéno.

7. Utilisation selon l'une quelconque des revendications 1-6, **caractérisée en ce que** R³ est choisi dans le groupe formé par phénylalkyle et naphtyle, substitués, chacun indépendamment, par au moins un substituant choisi dans le groupe formé par halogéno, alkyloxycarbonyle, hydroxy, alkyloxy et dialkylaminocarbonyle.

8. Utilisation selon la revendication 1, dans laquelle A=B représente C=O ou SO₂, R¹ représente alkyloxy, alkyloxyalkyle, Ar ou NR⁹R¹⁰, où R⁹ et R¹⁰ représentent, chacun indépendamment, hydrogène ou Ar ; ou A=B et R¹ ensemble forment un radical benzoxazolyle ; p représente zéro, R³ représente benzyle éventuellement substitué par hydroxy ou alkyloxycarbonyle et R⁴ et R⁵ représentent chacun hydrogène.

9. Utilisation selon la revendication 1, dans laquelle le composé est choisi dans le groupe formé par
le benzoate de 4-[[2-(1-benzoyl-4-phényl-4-pipéridinyl)-1H-imidazol-1-yl]méthyl]-méthyle ;
la 1-éthoxycarbonyl-4-phényl-4-[1-(1-phényléthyl)-1H-imidazol-2-yl]-pipéridine ;
le benzoate de 4-[[2-[1-(2-benzoxazolyl)-4-phényl-4-pipéridinyl]-1H-imidazol-1-yl]méthyl]-méthyle ;
la 1-benzoyl-4-phényl-4-[1-(phénylméthyl)-1H-imidazol-2-yl]-pipéridine ;
la 1-benzoyl-4-phényl-4-[1-(1-phényléthyl)-1H-imidazol-2-yl]-pipéridine ;
le N,4-diphényl-4-[1-(phénylméthyl)-1H-imidazol-2-yl]-1-pipéridine-sulfonamide ;
la 1-éthoxycarbonyl-4-phényl-4-[1-(phénylméthyl)-1H-imidazol-2-yl]-pipéridine ;
la 1-(méthoxyacétyl)-4-phényl-4-[1-(1-phényléthyl)-1H-imidazol-2-yl]-pipéridine ;
la [4-(1-benzyl-1H-imidazol-2-yl)-4-phényl-pipéridin-1-yl]-(3,5-diméthyl-phényl)-méthanone ;
le benzoate de 4-{2-[1-(2-méthoxy-acétyl)-4-phényl-pipéridin-4-yl]-imidazol-1-ylméthyl}-méthyle ;
la 4-(1-benzyl-1H-imidazol-2-yl)-4-phényl-1-thiazol-2-yl-pipéridine ;
le 2-{4-phényl-4-[1-(1-phényl-éthyl)-1H-imidazol-2-yl]-pipéridin-1-yl}-benzo-oxazole ;
la 1-[4-(1-benzyl-1H-imidazol-2-yl)-4-phényl-pipéridin-1-yl]-2-méthoxyéthanone ; et
la 2-[4-(1-benzyl-1H-imidazol-2-yl)-4-phényl-pipéridin-1-yl]-pyrimidine.

10. Utilisation selon l'une quelconque des revendications 1-9, **caractérisée en ce que** le trouble du système nerveux central est un trouble de dépression et/ou d'anxiété.

11. Utilisation selon l'une quelconque des revendications 1-10, **caractérisée en ce que** les composés selon la formule (I), les sels d'addition d'acide ou de base pharmaceutiquement acceptables de ceux-ci, les formes stéréochimiquement isomères de ceux-ci, les formes tautomères de ceux-ci et les formes N-oxydes de ceux-ci sont co-administrés avec d'autres antidépresseurs, anxiolytiques et/ou antipsychotiques.

12. Utilisation selon la revendication 11, en ce que les composés selon la formule (I), les sels d'addition d'acide ou de base pharmaceutiquement acceptables de ceux-ci, les formes stéréochimiquement isomères de ceux-ci, les formes tautomères de ceux-ci et les formes N-oxydes de ceux-ci et d'autres agents peuvent être présents sous forme d'une préparation combinée destinée à une utilisation simultanée, séparée ou séquentielle.
